# EUROPEAN PATENT APPLICATION

(11) **EP 4 089 093 A1**
(43) Date of publication of application: **16.11.2022**
(21) Application number: 21173663.2
(22) Date of filing: 12.05.2021
(51) Int. Cl.: C07D 471/06, A61P 35/00, A61K 31/5025

(54) **RADIOSYNTHESIS OF [18F] TALAZOPARIB**

(71) Applicant: Eberhard Karls Universität Tübingen Medizinische Fakultät, 72074 Tübingen (DE)
(72) Inventor: Bowden, Gregory David, 72074 Tübingen (DE); Maurer, Andreas, 72076 Tübingen (DE); Stotz, Sophie, 72074 Tübingen (DE); Kinzler, Paul Johannes Oskar Maria, 72160 Horb am Neckar (DE); Lämmerhofer, Michael, 72070 Tübingen (DE); Zender, Lars, 72076 Tübingen (DE); Pichler, Bernd, 85298 Scheyern (DE)
(74) Representative: Witte, Weller & Partner Patentanwälte mbB

(57) **Abstract**

The present invention relates to the synthesis of Poly (ADP ribose) polymerase (PARP) inhibitors and particularly to the radiosynthesis of PARP 1 inhibitors, more particularly to the synthesis of [¹⁸F] talazoparib.

## Description

### FIELD OF THE INVENTION

The present invention relates to the synthesis of Poly (ADP ribose) polymerase (PARP) inhibitors and particularly to the radiosynthesis of PARP 1 inhibitors, more particularly to the synthesis of [¹⁸F] talazoparib.

### BACKGROUND OF THE INVENTION

In the course of the current developments of personalized cancer treatment Poly (ADP ribose) polymerase 1 (PARP1) has become an important target. This has spurred the development of several highly potent PARP inhibitors that induce synthetic lethality in malignant tumors that lack the homologous recombination (HR) DNA repair pathway. PARP overexpression in several tumor entities has also made the enzyme a valuable biomarker for optical and nuclear imaging techniques to detect malignant lesions and aid surgical excision of difficult-to-detect cancer entities. Non-invasive imaging techniques such as positron emission tomography (PET) are used to track the fate of a radioactively labeled molecule *in vivo;* thus, aiding diagnosis and therapy surveillance Quantification of PARP1 expression is thought to be predictive of tumor malignancy, as its overexpression is correlated with a clinical poor prognosis.

The PARP enzyme family consists of 17 members of which PARP1 is the most abundant and best-characterized. It senses DNA single-strand breaks (SSBs) by its zinc finger domains and initiates their repair by poly (ADP ribosylation) (PARylation) of target enzymes. PARP1, PARP2 and PARP5a and b (tankyrase 1 and 2) are capable of PAR chain formation while the remaining PARP enzymes can produce mono (ADP ribose) ((MARyl)ation) or are enzymatically inactive.

Although the regulatory domains of PARP enzymes differ drastically, they share the same conserved catalytic domain and use nicotinamide adenine dinucleotide (NAD⁺) as the substrate for PARylation. PARP inhibitors structurally mimic NAD⁺, thus blocking the catalytic domain and escalating the SSBs to double-strand breaks (DSBs) that can be repaired by alternative DNA repair mechanisms as HR in healthy tissue.

Besides their catalytic inhibition, some PARP inhibitors are able to trap the PARP enzyme on the damaged DNA site to a certain extent which leads to replication fork collapse.

Within the last decade, numerous PARP inhibitors were approved by the Food and Drug Administration (FDA): olaparib (2014), rucaparib (2016), niraparib (2017) and talazoparib (2018). Mainly used for maintenance or combination therapies with DNA damaging agents like cisplatin in selected cancer entities, ongoing studies explore the potential of PARP inhibitors as monotherapy in HR-potent tumors.

As explanation for the different cytotoxic efficacy of PARP inhibitors, the drastic difference in the ability to trap PARP1 on the DNA is commonly agreed to. Talazoparib is so far considered the most potent PARP1 trapping agent, featuring the lowest IC₅₀ of 0.56 nM towards PARP1 (olaparib: 5 nM, rucaparib: 0.65 nM, niraparib: 3.8 nM) and similar efficacy in much lower doses. Structurally, the molecule exhibits two distinct chiral centers, and only the (8S, 9R)-enantiomer (talazoparib) demonstrates high potency while the (8R, 9S)-enantiomer (LT-674) is less active by several orders of magnitude. The exact molecular mechanism of PARP trapping and its linkage to catalytic inhibition remains unclear, although it is proposed that the bulky structure and stereochemistry of talazoparib contributes to its high potency.

Outstanding examples for the development of PARP1-targeted imaging probes are PARPi-FL, an fluorescently labeled olaparib analogue, together with its radioactive sibling [¹⁸F] PARPi, and the in-human PARP radiotracer [¹⁸F] FTT that have been used in the detection and delineation of oral cancer, detection of brain, pancreatic and liver cancer, and target engagement imaging of different PARP inhibitors.

Although several PARP inhibitor-based imaging agents are successfully established in preclinical and clinical settings, e.g. the olaparib-based PARPi-FL, a radiotracer based on talazoparib or a talazoparib derivative is not known.

The synthesis of radiotracers needs to fulfil several demands with respect to handling and suitability for use in living entities. On the one hand, the radiotracer needs to exhibit a retention time and half life time that are short enough for enabling the use *in vivo* with an acceptable radiation exposure to the patient. Furthermore, the half-life has to be long enough for enabling preparation and administration without excessive decay of the radiotracer.

In addition, an optimized synthesis with a limited number of method steps is required for reducing environmental risks as well as health hazards for the persons handling the radiochemicals, such as the chemist preparing and/or purifying the radiolabeled talazoparib or talazoparib derivatives, and the medical practitioner handling and administering said compounds. In general, it is desirable that the synthesis is uncomplicated, automatable, fast, reliable and offers high yields.

It is therefore an objective of the present invention to provide radiolabeled talazoparib or talazoparib derivatives that fulfil the above mentioned requirements. Another objective of the present invention resides in the provision of easy accessible precursors for synthesizing the radiolabeled talazoparib or talazoparib derivatives. Still another objective resides in the provision of a corresponding method for providing talazoparib or talazoparib derivatives starting from said easily accessible precursors.

### SUMMARY OF THE INVENTION

In course of the studies leading to the present invention, it has been found that [¹⁸F] radiolabeling is particularly suitable for fulfilling the above mentioned objectives.

According to a preferred embodiment of the present invention, a compound of the formula 10a and/or 10b 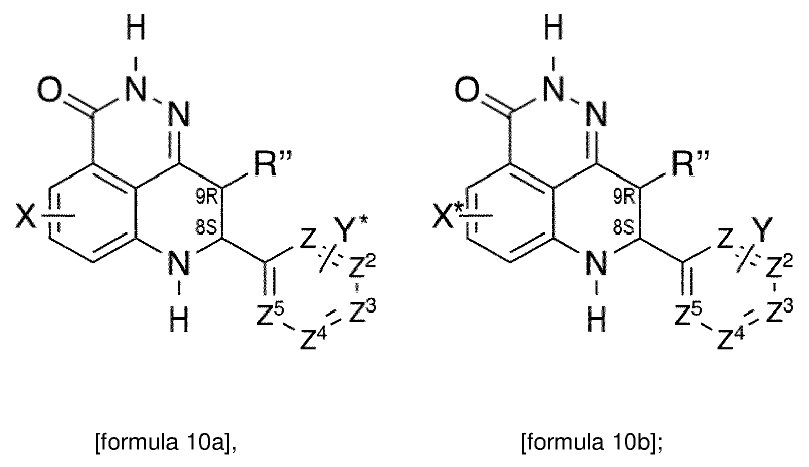 or a precursor of the formula 5c a is provided 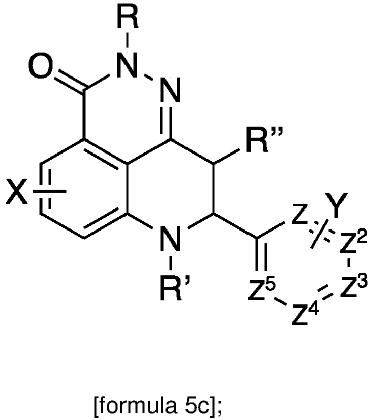 wherein
R and R' are amine protecting groups;
R" is a heteroaromatic or an aromatic residue of a molecular weight of 120 g/mol or less;
X and Y are independently selected from the group consisting of H, one or more leaving groups for [¹⁸F] fluorination, and a saturated or unsaturated alkyl residue of a molecular weight of 80 g/mol or less, provided that at least one leaving group for [¹⁸F] fluorination is present;
X* and Y* are independently selected from the group consisting of H, [¹⁸F], one or more leaving groups for [¹⁸F] fluorination and a saturated or unsaturated alkyl residue of a molecular weight of 80 g/mol or less, provided that at least one [¹⁸F] is present;
z and Z²⁻⁵ are independently selected from the group consisting of C and N; and
an enantiomeric ratio (er) at a position C8 and/or a position C9 of the precursor of the formula 5c is ≥ 1.

According to another preferred embodiment of the present invention, a method for producing a compound of the formula 10a and/or 10b 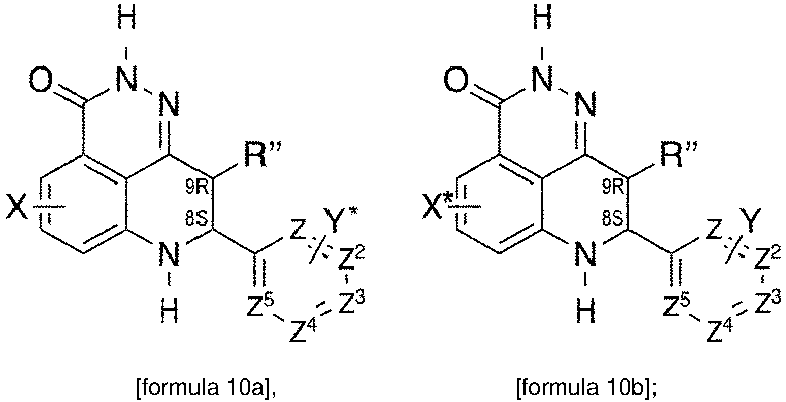 is provided.

The method comprises, preferably consists of, the steps of:
(A) providing a precursor of the formula 5c 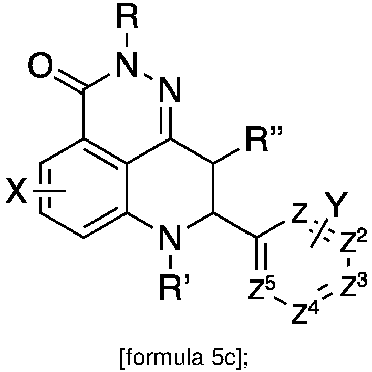
(B) reacting the precursor of the formula 5c with an [¹⁸F] radiofluorination agent to obtain a [¹⁸F] fluorinated compound of the formula 8a and/or 8b 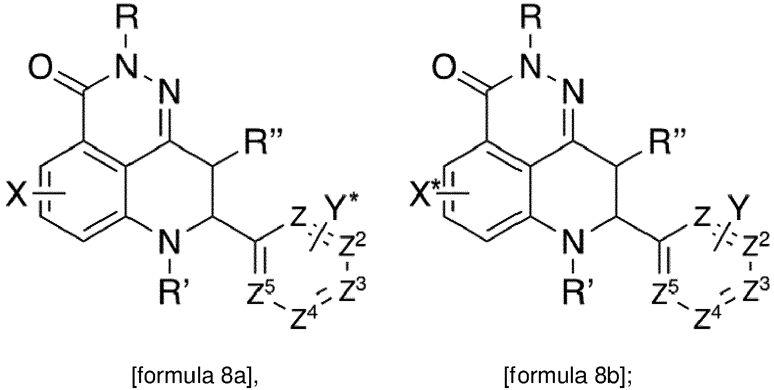
(C) de-protecting the [¹⁸F] fluorinated compound of the formula 8a and/or 8b to obtain a compound of the formula 9a and/or 9b 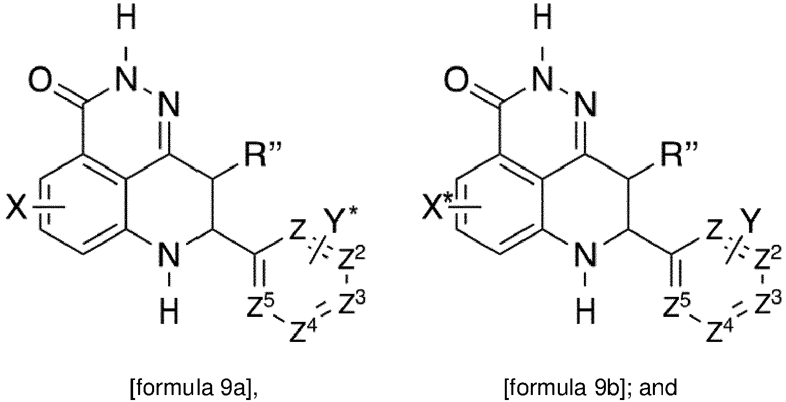
(D) subjecting the compound of the formula 9a and/or 9b to purification and optionally enantiomeric resolution to obtain the compound of the formula 10a and/or 10b,
   wherein
   R and R' are amine protecting groups;
   R" is a heteroaromatic or an aromatic residue of a molecular weight of 120 g/mol or less;
   X and Y are selected from the group consisting of H, one or more leaving groups for [¹⁸F] fluorination, or a saturated or unsaturated alkyl residue of a molecular weight of 80 g/mol or less, provided that at least one leaving group for [¹⁸F] fluorination is present;
   X* and Y* are independently selected from the group consisting of H, [¹⁸F], one or more leaving groups for [¹⁸F] fluorination and a saturated or unsaturated alkyl residue of a molecular weight of 80 g/mol or less, provided that at least one [¹⁸F] is present;
   z and Z²⁻⁵ are independently selected from the group consisting of C and N; an enantiomeric ratio (er) at a position C8 and/or a position C9 of the precursor is ≥ 1.

### DETAILED DESCRIPTION

The term "talazoparib" designates the compound (8*S*,9*R*)-5-Fluoro-8-(4-fluorophenyl)-2,7,8,9-tetrahydro-9-(1-methyl-1*H*-1,2,4-triazol-5-yl)-3*H*-pyrido[4,3,2-de]phthalazin-3-one. Talazoparib is the biologically active enantiomer and is also known as BMN 673 or Talzenna. The biologically inactive (8R, 9S) enantiomer of talazoparib is also designated as "LT-674".

The expression "[¹⁸F] talazoparib" as used herein refers to talazoparib with one or both fluorine atoms substituted by ¹⁸F, i.e. the compounds (8S,9R)-5-[¹⁸F]Fluoro-8-(4-fluorophenyl)-2,7,8,9-tetrahydro-9-(1-methyl-1*H*-1,2,4-triazol-5-yl)-3*H-*pyrido[4,3,2-de]phthalazin-3-one, (8*S*,9*R*)-5-Fluoro-8-(4--[¹⁸F]fluorophenyl)-2,7,8,9-tetrahydro-9-(1-methyl-1*H*-1,2,4-triazol-5-yl)-3*H*-pyrido[4,3,2-*de*]phthalazin-3-one, and (8*S*,9*R*)-5-[¹⁸F]Fluoro-8-(4-[¹⁸F]fluorophenyl)-2,7,8,9-tetrahydro-9-(1-methyl-1H-1,2,4-triazol-5-yl)-3*H*-pyrido[4,3,2-de]phthalazin-3-one. The compounds of formula 10a and/or 10b include the above mentioned compounds and the remaining compounds of formula 10a and/or10b are also referred to "[¹⁸F] talazoparib derivatives".

It is preferred that the compounds of formula 10a and/or 10b have a single ¹⁸F radiolabel, preferably ¹⁸F at the position C5 or z³, more preferably ¹⁸F at the position z³. Radiolabeling at these positions is expected to have no disadvantageous effects on the retention time and biological activity of the molecules.

Amine protecting groups are well known to the skilled person.

In general, a "protecting group" or a "protective group" is a reversibly formed derivative of an existing functional group in a molecule. The protective group is temporarily attached to decrease reactivity so that the protected functional group does not react under synthetic conditions to which the molecule is subjected in one or more subsequent steps. For instance, protecting an amine as a carbamate enables other functional groups to undergo selective reactions with electrophiles whereby the carbamate (protected amino group) is left intact. This involves two additional synthetic steps: the step to form the protected intermediate and a deprotection once the additional selective synthetic steps have been completed.

The nature of the protective group must be chosen carefully to ensure adequate stability throughout all the intermediary synthesis steps. Furthermore, the conditions for the protection and deprotection steps and the nature of the protective group itself must not interfere with other functional groups present in the molecule.

If more than one functional group of the same type is present in a molecule, subtle differences in reactivity - for example caused by steric effects - can help to achieve the selective protection of just one functional group while another such functional group remains unprotected. Alternatively, a second such functional group could be protected with a different protecting group that has a different reactivity profile. Another opportunity is to build a larger molecule from subunits in which similar or identical functional groups have been differently protected beforehand. For example, a Boc-protected amino group can be deprotected in acidic media, whereas a Fmoc-protected amino group can be deprotected under basic conditions. The presence of both protective groups in the same molecule therefore enables selective deprotection of one protected amino group for a further reaction while the second protected amino group remains untouched. This is referred to as an orthogonal protecting group strategy.

Not only is selectivity important, but the yields for the protection and deprotection steps must be high to avoid making the reaction sequence inefficient.

Protection/ deprotection of amines may be performed by any of carbobenzyloxy group/ hydrogenolysis, *p*-methoxybenzyl carbonyl (Moz or MeOZ) group/ hydrogenolysis; *tert*-butyloxycarbonyl (BOC) group/ strong acid, such as HCl or CF₃COOH), and/or by heating to >80°C; 9-fluorenylmethyloxycarbonyl (Fmoc)/ base, such as piperidine; acetyl (Ac) group/ base, such as methylamine benzoyl (Bz) group/ base, such as methylamine; benzyl (Bn) group/ hydrogenolysis; carbamate group/ acid and mild heating; p-methoxybenzyl (PMB) group/ hydrogenolysis; 3,4-Dimethoxybenzyl (DMPM) group/ hydrogenolysis, p-Methoxyphenyl (PMP) group/ ammonium cerium(IV) nitrate (CAN); tosyl (Ts) group/ concentrated acid, such as HBr, H₂SO₄, and/or strong reducing agents, such as sodium in liquid ammonia; trichloroethyl chloroformate (troc) group/ Zn insertion in presence of acetic acid; sulfonamide (Nos) group/ samarium iodide or tributyltin hydride.

In course of the studies leading to the present invention, it has been found that suitable amine protecting groups amine protecting group are trimethylsilylethoxymethyl, *t*-butyl carbamate, benzyloxy carbamate, and methoxymethyl acetal.

The term "heteroaromatic residue" pertains to residues that encompass an unsaturated cyclic compounds having 5 or 6 atoms that are selected from C, N, O, or S; preferably C or N. More preferred are saturated cyclic compounds having 4 C and 1 N atoms, 3 C and 2 N atoms, 2 C and 3 N atoms, 5 C and 1 N atoms, 4 C and 2 N atoms, or 3 C and 3 N atoms. Examples of preferred heteroaromatic residues involve me-1,2,4-triazol-5-yl, 4-me-1,2,4-triazol-3-yl, 4-pyridyl, 3-pyridyl, 2-pyridyl, 1-me-imidazol-2-yl, and 1-me-pyrazol-5-yl.

An "aromatic residue" as used herein encompasses an unsaturated cyclic compound having 6 C-atoms. The "aromatic residue" may exhibit one or more functional groups such as methyl or ethyl. A preferred aromatic residue is phenyl.

The molecular weight of the heteroaromatic residue or aromatic residue is 120 g/mol or less, preferably 110 g/mol or less, 100 g/mol or less, or 90 g/mol or less.

X and Y are selected from the group consisting of H, one or more leaving groups for [¹⁸F] fluorination, or a saturated or unsaturated alkyl residue of a molecular weight of 80 g/mol or less, such as 70 g/mol or less, 60 g/mol or less, 50 g/mol or less, 40 g/mol or less, or 30 g/mol or less, provided that at least one leaving group for [¹⁸F] fluorination is present.

The molecular sizes of the heteroaromatic residue, aromatic residue as well as the saturated or unsaturated alkyl residue ensures that that the [¹⁸F] talazoparib derivatives do not exhibit a sterical hindrance that negatively affects or even impedes the biological activity. In addition or alternatively, the retention time is not negatively affected, i.e. the retention may be similar to that of talazoparib.

Leaving groups for [¹⁸F] fluorination are well known in the art and includes any leaving group susceptible to nucleophilic fluorination, such as Cl, Br, or I. Exemplary reactions involve the reactions of corresponding bromonium salts or iodonium salts with the cyclotron- produced [¹⁸F]-potassium fluoride in the presence of a phase-transfer reagent, such as Kryptofix-222.

It will be appreciated that other residues, such as nitro, trialkylamine, halogen, mesylate, tosylate, or triflate, may be susceptible to nucleophilic fluorination as well depending on the molecules structures, particularly the presence of electron withdrawing groups or atoms that are close to the leaving group for [¹⁸F] fluorination, i.e. that are located within the same ring structure as the leaving group for [¹⁸F] fluorination.

It will be understood that not all leaving groups for ¹⁸F fluorination are substituted by ¹⁸F in course of the present method. Rather one or more leaving groups, such as one, two, or three leaving groups, may remain in the compound of formula 10a and/or 10b provided that at least one leaving groups for ¹⁸F fluorination is in fact converted to ¹⁸F.

It has been found in course of studies leading to the present invention that the concepts of nucleophilic fluorination are stretched to its limits in context with the present compounds. The reason essentially resides in the presence of nitrogen based heterocycles, which are believed to complex the copper mediator and forming unreactive copper species that inhibit radiosynthesis performance. It is therefore assumed that particularly suitable leaving groups [¹⁸F] fluorination include boronic acids, boronic acid esters, iodonium salts, iodonium ylids, trialkylstannanes, a nitro group, trialkyl ammonium salts, and sulfonate esters, such as tosylate (OTs), mesylate (OMs), nosylate (ONs), brosylate (OBs), or trifluoromethanesulfonate (OTf).

The present invention is based on the finding that an accessible radiosynthesis of [¹⁸F] talazoparib or [¹⁸F] talazoparib derivatives has become possible because of the recent advances in radiochemical methodology such as copper mediated radiofluorination chemistry (CMRF) (Wright, J. S.; Kaur, T.; Preshlock, S.; Tanzey, S. S.; Winton, W. P.; Sharninghausen, L. S.; Wiesner, N.; Brooks, A. F.; Sanford, M. S.; Scott, P. J. H., Copper-Mediated Late-stage Radiofluorination: Five Years of Impact on Pre-clinical and Clinical PET Imaging. Clin TranslImaging 2020, 8(3), 167-206. Tredwell, M.; Preshlock, S. M.; Taylor, N. J.; Gruber, S.; Huiban, M.; Passchier, J.; Mercier, J.; Genicot, C.; Gouverneur, V., A general copper-mediated nucleophilic 18F fluorination of arenes. Angew Chem Int Ed Eng/2014, 53 (30), 7751-5. Makaravage, K. J.; Brooks, A. F.; Mossine, A. V.; Sanford, M. S.; Scott, P. J. H., Copper-Mediated Radiofluorination of Arylstannanes with [(18)F]KF. Org Lett2016, 18 (20), 5440-5443. Mossine, A. V.; Brooks, A. F.; Makaravage, K. J.; Miller, J. M.; Ichiishi, N.; Sanford, M. S.; Scott, P. J., Synthesis of [18F]Arenes via the Copper-Mediated [18F]Fluorination of Boronic Acids. Org Lett 2015, 17 (23), 5780-3).

The present inventors have explored this methodology through the use of the Design of Experiments (DoE) approach to reaction optimization and to lay the foundation of a radiotracer development pipeline using CMRF chemistry (Bowden, G. D.; Pichler, B. J.; Maurer, A., A Design of Experiments (DoE) Approach Accelerates the Optimization of Copper-Mediated (18)F-Fluorination Reactions of Arylstannanes. Sci Rep 2019, 9(1), 11370.

DoE aids in establishing reliable and robust radiosynthesis from the onset of the tracer development process and, in combination with known de-risking strategies described by Taylor *et al.,* this approach can be used expedite the development of novel radiotracers (Taylor, N. J.; Emer, E.; Preshlock, S.; Schedler, M.; Tredwell, M.; Verhoog, S.; Mercier, J.; Genicot, C.; Gouverneur, V., Derisking the Cu-Mediated (18)F-Fluorination of Heterocyclic Positron Emission Tomography Radioligands. J Am Chem Soc 2017, 139 (24), 8267-8276). The present inventors successfully applied a DoE based tracer development workflow to the challenging radiosynthetic problem of [¹⁸F] talazoparib and its derivatives. Here, the first synthesis of [¹⁸F] talazoparib and its derivatives is presented as a second-generation PARP radiotracer together with a preliminary *in vitro* evaluation of [¹⁸F] talazoparib.

The synthesis of chiral [¹⁸F] talazoparib and [¹⁸F] talazoparib derivatives posed a complex radiosynthetic challenge with two possible approaches. A first approach is to employ an enantiomerically pure precursor in the radiosynthesis. However, for an as of yet uncharacterized radiotracer, chiral resolution of the precursor is usually costly and time consuming and that there was a risk of the chiral precursor racemizing during the synthesis. For this reason, said approach is generally less preferred.

Another approach first involves the production and purification of the racemic radiotracer, followed by enantiomeric separation, isolation and formulation of the active enantiomer as depicted in the present method. Therefore, as the radiochemical yield would be reduced by half (assuming a 1:1 ratio of enantiomers), the radiosynthesis would need to be carefully optimized using DoE for maximum yield to ensure an adequate and reliable synthesis output for biological studies. The two possible regions for isotopic ¹⁸F radiolabeling, shown by X* and Y* in the present formulas, are both situated on aromatic rings. It has been found that these premises exclude the use of traditional S_{N}Ar radiofluorination and require the use of more recently developed methodologies such as CMRF chemistry. However, the proposed radiotracer contains multiple heteroaromatic nitrogen atoms that have been shown to have detrimental effects on CMRF based radiosynthesis. These moieties are thought to complex with the copper mediator, forming unreactive copper species that inhibit radiosynthesis performance.

For this reason, the present inventors set out to develop a protecting group strategy that would allow for maximal radiosynthetic performance while still affording an easily accessible, convenient to use, and shelf-stable precursor. As thus, an adequately protected precursor would have to be developed.

An exemplified synthetic route of the precursor of formula 5c and the compounds of formula 10a and 10b is shown hereinafter. Even though this synthetic route is preferred, it shall not be construed as limiting.

The present inventors adapted the synthesis of the precursor from the synthetic route employed by Wang *et al.* for the synthesis of talazoparib (Wang, B.; Chu, D.; Feng, Y.; Shen, Y.; Aoyagi-Scharber, M.; Post, L. E., Discovery and Characterization of (8S,9R)-5-Fluoro-8-(4-fluorophenyl)-9-(1-methyl-1H-1,2,4-triazol-5-yl)-2,7,8,9-te trahydro-3H-pyrido[4,3,2-de]phthalazin-3-one (BMN 673, talazoparib), a Novel, Highly Potent, and Orally Efficacious Poly(ADP-ribose) Polymerase-1/2 Inhibitor, as an Anticancer Agent. J Med Chem 2016, 59 (1), 335-57). It will be appreciated, however, that also other synthetic routes may be used as starting points for the provision of the precursor of formula 5c.

In particular, 1-methyl-1H-1,2,4-triazole was formylated to 1 via the published procedure (Albrecht, Brian K.; Bauer, David; Bellon, Steven; Bode, Christiane M.; Booker, Shon; Boezio, Alessandro; Choquette, Deborah; D'Amico, Derin; Harmange, Jean-Christophe; Hirai, Satoko; Hungate, Randall W.; Kim, Tae-Seong; Lewis, Richard T.; Liu, Longbin; Lohman, Julia; Norman, Mark H.; Potashman, Michelle; Siegmund, Aaron C.; Springer, Stephanie K.; Stec, Markian; Xi, Ning; Yang, Kevin, (2009) FUSED HETEROCYCLIC DERIVATIVES AND METHODS OF USE, WO 2009/091374). **1** was then condensed with commercially available 6-fluoro-4-nitroisobenzofuran-1(3H)-one in THF in the presence of NEt₃ and Ac₂O to afford **2** in a 53% yield. The lactone **2** was opened to give the keto-ester derivative **3** by warming **2** in methanol with a catalytic amount of acetic acid. In the original synthesis, **3** was then reacted with 4-fluorobenzaldehyde in THF and methanol via reductive cyclization driven by TiCl₃ to afford **4b,** which was cyclized with hydrazine monohydrate to afford (±)talazoparib (**5b**), which was to be used as reference compound. Reacting **3** with 4-bromobenzaldehyde via the same two-step procedure afforded the derivatizable bromide **5a.**

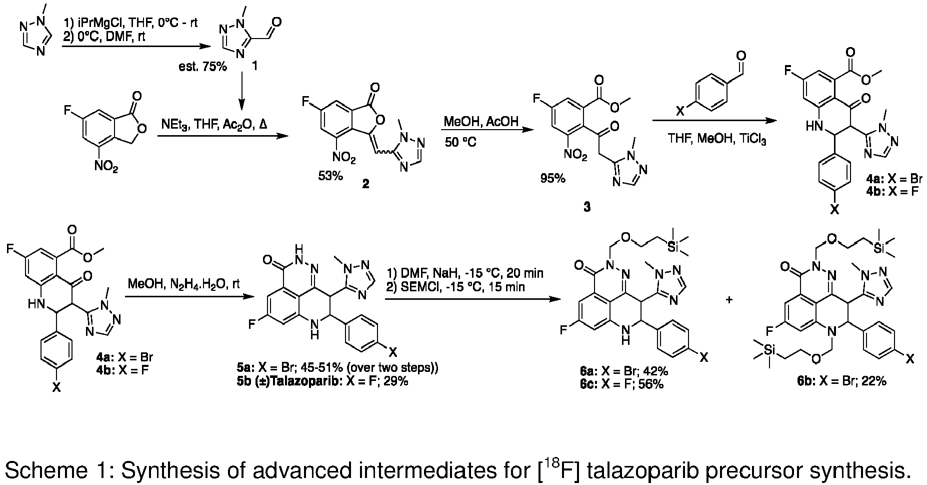

Alternative possibilities for the synthesis of the precursor of the formula 5c as well as the compounds of the 10a and 10b are derivable from scheme 1. The present inventors have found that reacting keto-ester derivative **3** with aromatic aldehydes with different substituents than 4-bromobenzaldehydes influences the overall structure of the compound of the formula 10a. The differently substituted aromatic aldehydes may involve benzaldehyde derivatives, wherein z and z²⁻⁵ are independently selected from the group consisting of C and N, and Y* is selected from the group consisting of H, [¹⁸F], one or more leaving groups for [¹⁸F] fluorination and a saturated or unsaturated alkyl residue of a molecular weight of 80 g/mol or less, provided that at least one [¹⁸F] is present.

It has been furthermore found that reacting **1** with a 4-nitroisobenzofuran-1 (3H)-one exhibiting the residue Y* at one or more of the positions C4, C5, and C6 affects the overall structure of the compound of the formula 10b. It will be appreciated that the provision of differently substituted aromatic aldehydes than 4-bromobenzaldehydes as well as a 4-nitroisobenzofuran-1(3H)-one that exhibits the residue Y* at one or more of the positions C4, C5, and C6 is well known to the skilled person.

Employing an aromatic aldehyde that is differently substituted at the phenyl moiety, particularly at any of the positions z²⁻⁵, than 4-bromobenzaldehyde according to scheme 1and/or a 4-nitroisobenzofuran-1(3H)-one that is differently substituted at one or more of the positions C4, C5, and C6 than 6-fluoro-4-nitroisobenzofuran-1(3H)-one according to scheme 1 results in the generation of compounds that are different from (±) talazoparib, namely a precursor of the formula 5c.

A protecting group strategy using the trimethylsilylethoxymethyl ether (SEM) protecting group was employed to the compound **5a,** which was protected with trimethylsilylethoxymethyl chloride (SEMCI) using sodium hydride in DMF at 15 °C. These conditions afforded both the mono- (**6a**) and di-SEM (**6b**) protected bromide derivatives. It is clear, however, that the protecting group strategy is not limited to the disclosed compounds.

Compounds **6a** and **6b** were then further derivatized to yield several possible precursors for radiolabeling (Scheme 2). The protection of **5a** favored the formation **6a** over **6b** (ca. 3:1), even under conditions designed to drive the formation of the di-SEM protected derivative. Compounds **6a** and **6b** were converted to their corresponding boronic acid pinacol esters under Miyaura borylation conditions in DMF with KOAc, bis(pinacolato)diboron, and Pd(dppf)Cl₂ as the catalyst (Scheme 2). This afforded the compounds **7a** and **7b** in good to excellent yields. **7a** was purified through recrystallization a shelf-stable and convenient-to-handle white crystalline solid. **7b** however, was purified through chromatography and was much harder to obtain in the high purities required for reliable radiochemistry. Despite being of high chromatographic purity (HPLC-MS), samples of **7b** displayed a complex NMR spectrum, indicative of the presence of multiple conformation isomers. CMRF chemistry has also been well established with aryl stannanes precursors and the present inventors have investigated this variation of the CMRF reaction extensively in our previous work. Thus, the analogous stannylation of **6a** was carried with bis tributyltin to afford the stannyl precursor **7c** as an amorphous glass. It will be readiliy understood that the above mentioned protecting and deprotecting agents are merely examples and other protecting and deprotecting agents may be used as well.

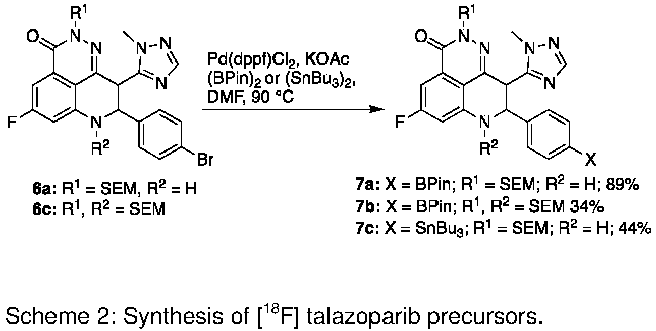

According to an embodiment of the present invention, the amine protecting group at R and R' is independently selected from the group consisting of trimethylsilylethoxymethyl, *t*-Butyl carbamate, benzyloxy carbamate, and methoxymethyl acetal. As noted above protection and deprotection strategies for amines are known to the skilled person.

A protecting group strategy with trimethylsilylethoxymethyl ether (SEM) protecting group involves e.g. the use of a trimethylsilylethoxymethyl halide, such as trimethylsilylethoxymethyl chloride (SEMCI), using a base and a solvent at reduced temperatures, such as sodium hydride in DMF at 0 °C. It is clear, however, that the protecting group strategy is not limited to the disclosed compounds.

Protection and deprotection are performed according to the skilled persons knowledge. Deprotection may involve, for instance, treatment with a concentrated acid, such as HCl or HNO₃ with a concentration of 1 mol/l or more, such as 3 mol/l or more, and/or at elevated temperatures of e.g. 80°C or more, such as 90°C, or more or 100°C or more for a time period of e.g. 10 min. or more, such as 15 min. or more. Suitable deprotection conditions involve e.g. 6 M HCI at 120 °C for 15 min.

According to another embodiment of the present invention, the heteroaromatic residue is selected from the group consisting of me-1,2,4-triazol-5-yl, 4-me-1,2,4-triazol-3-yl, 4-pyridyl, 3-pyridyl, 2-pyridyl, 1-me-imidazol-2-yl, 1-Me-pyrazol-5-yl, wherein the aromatic residue is phenyl.

According to still another embodiment of the present invention, z is N and z²⁻⁵ are C; z² is N and z, z³⁻⁵ are C; or z³ is N and z, z², z⁴⁻⁵ are C. Alternatively, z and z² are N and z³⁻⁵ are C, z and z³ are N and z², z⁴⁻⁵ are C, z² and z³ are N and z, z⁴⁻⁵ are C, or z² and z⁴ are N and z¹, z³, and z⁵ are C. It is preferred that at z and z²⁻⁵ involve not more than two N atoms, preferable a single N atom, due to the impact on CMRF radiosynthesis.

According to an embodiment of the present invention, the leaving group for [¹⁸F] fluorination is selected from the group consisting of boronic acid, boronic acid ester, iodonium salt, iodonium ylid, trialkylstannane, nitro, trialkyl ammonium salts, and sulfonate esters, such as OTs, OMs, ONs, OBs, or OTf.

According to another embodiment of the present invention, the radiofluorination agent is a nucleophilic [¹⁸F] radiofluorination agent, preferably wherein the nucleophilic [¹⁸F] radiofluorination agent is [¹⁸F] TBAF.

According to still another embodiment of the present invention, the compound of the formula 10a is [¹⁸F] talazoparib, and/or the compound of formula 10b is (8S,9R)-5-[¹⁸F]-8-(4-fluorophenyl)-2,7,8,9-tetrahydro-9-(1-methyl-1H-1 ,2,4-triazol-5-yl)-3H-pyrido[4,3,2-de]phthalazin-3-one.

According to an embodiment of the present invention, the step of preparing the precursor of the formula 5c by (i) comprises, preferably consists of, reacting a compound of the formula 5d and/or 5e 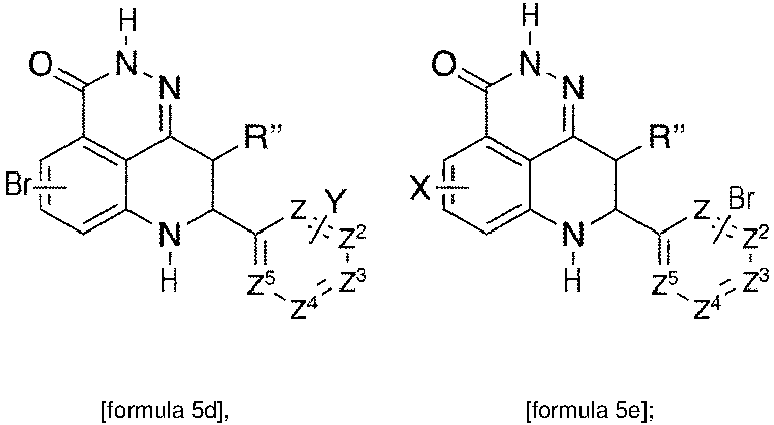 with a trimethylsilylethoxymethyl salt, preferably trimethylsilylethoxymethyl chloride; and
(ii) derivatizing to the corresponding boronic acid pinacol esters of the formula 7d and/or 7e 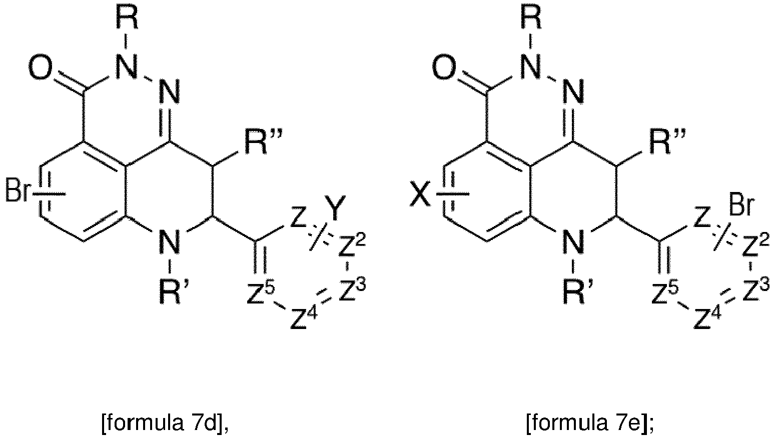 wherein R' is trimethylsilylethoxymethyl, R" is trimethylsilylethoxymethyl or H, and the leaving group for [¹⁸F] fluorination is boronic acid pinacol ester or tributylstannane.

According to another embodiment of the present invention, the compound of the formula 5d is (±)-5-fluoro-8-(4-halophenyl)-2,7,8,9-tetrahydro-9-(1-methyl-1*H*-1,2,4-triazol-5-yl)-3*H*-pyrido[4,3,2-*de*]phthalazin-3-one, and/or the compound of the formula 5e is (±)-5-halo-8-(4-fluorophenyl)-2,7,8,9-tetrahydro-9-(1-methyl-1*H*-1,2,4-triazol-5-yl)-3*H*-pyrido[4,3,2-de]phthalazin-3-one, wherein halo is bromine or iodine, preferably bromine.

According to still another embodiment of the present invention, the purification of the compound of the formula 9a and/or formula 9b of step (D) includes subjecting the compound of the formula 9a and/or formula 9b to solid phase extraction with a sorbent for polar analytes to obtain a purified compound of the formula 9a and/or formula 9b.

It is preferred that enantiomeric resolution of the compound of the formula 9a and/or formula 9b of step (D) includes subjecting the purified compound of the formula 9a and/or 9b to one or more of chiral NPLC, chiral SFC, and chiral HPLC, preferably chiral HPLC, to obtain the compound of the formula 10a and/or 10b.

With the precursors **7a-c** in hand, the present inventors set out to evaluate which precursor would provide the best radiosynthesis performance, while still being relatively easy to produce, shelf-stable, and convenient to work with. The compounds **7a, 7b,** and **7c** have shown acceptable results with **7a** being preferred. It will be clear, however, that the other compounds than the precursors with the formula **7a, b,** and **7c** may be used as well.

Any suitable solid phase extraction or purification method with a sorbent for polar or non-polar analytes, such as normal phase liquid chromatography (NPLC), supercritical fluid chromatography (SFC), or high performance liquid chromatography (HPLC) purfication, preferably chiral HPLC purification, may be employed to obtain a purified compound of the formula 9a and/or formula 9b.

Optimal chiral HPLC conditions were screened using racemic (±) talazoparib in combination with commercially obtained authentic samples of talazoparib and LT-674. Different HPLC columns may be used to resolve the two enantiomers under reverse-phase conditions according to the manufacturer's suggestion. Employed solvents are known to the skilled person and involve, for instance, 0.1% TFA in water: acetonitrile.

Different CHIRALPAK materials, such as CHIRALPAK IA, IB, and IC, may be employed. It has been found that CHIRALPAK IB packing material provides a good enantiomeric resolution at relatively low retention time. CHIRALPAK IB-N5 (5 µm) columns under reverse-phase conditions would thus be used for both semipreparative enantiomeric resolution of [¹⁸F] talazoparib, its derivatives and [¹⁸F]LT-674 and for chiral quality control (QC) analysis.

The enantiomeric resolution of the compound of the formula 9a and/or 9b may be avoided, i.e. is optional, in case of using a precursor compound that is already enantiomerically resolved, i.e. a precursor that exhibits the required configuration at positions corresponding to C8 and C9 of the compound of the formula 10a and/or 10b. For instance, the precursor of the formula 5c may already exhibit the required (8S,9R)-configuration. Alternatively, and preferably, precursors are employed, such as the precursor of the formula 5c, that are in (±)-configuration. In that case, the purification of the compound of the formula 9a and/or 9b of step (D) involves subjecting the purified compound of the formula 9a and/or 9b to one or more of chiral NPLC, chiral SFC, and chiral HPLC, preferably chiral HPLC, to obtain the compound of the formula 10a and/or 10b.

According to another embodiment of the present invention, the step (B) of reacting the precursor of the formula 5c with an [¹⁸F] radiofluorination agent includes copper mediated radiofluorination chemistry that is optimized with respect to the radiochemical yield of the (±)¹⁸F fluorinated compound of the formula 8a and/or 8b.

Optimization with respect to the radiochemical yield of a (±)¹⁸ F fluorinated derivative is preferably based on the assessment of a plurality of experiments that are carried out with the radioactive label. It is preferred that a design of experiments (DoE) study is performed according to standards and conditions that are known to the skilled person.

In this regard the use of DoE has been explored to solve complex radiochemical optimization problems and have developed a ¹⁸F processing method and workflow that is compatible with small scale DoE radiochemical experiments. The present inventors applied this workflow to a computer-generated D-optimal DoE study designed investigate the effects of the individual constituents, including the precursor (the precursor load), the reactant(s) (such as the copper-mediator load), solvent(s) (such as the pyridine load) and optional cosolvent(s) (the pyridine load in DMA) on reaction performance.

Such a DoE study requires a plurality of experiments using [¹⁸F] fluoride from cyclotron target washes, wherein each experiment involves the same reaction conditions with the proviso of the amounts of the individual constituents.

Outliers may be excluded according to predefined criteria. The remaining data may be used to fit a regression model with an acceptable goodness of model fit and goodness of model prediction, respectively. It will be appreciated that these steps are measures that are known to the skilled person. A response surface plot is generated from the adopted regression model and used for identifying optimal amounts of the individual constituents under the given reaction conditions.

The results of the optimized CMRF radiosynthesis are then translated onto available automated synthesis systems, such as a cassette-based system (e.g. the Elixys Flex/Chem) equipped with HPLC injection loops and HPLC columns, such as two semipreparative HPLC injection loops and up to 3 selectable HPLC columns (such as the Pure/Form module) or the Tracerlab FX N Pro that is a fixed fluid path system with a single HPLC injection loop and column.

The DoE optimized reaction mixture is reacted and treated with the above mentioned system.

According to still another embodiment of the present invention, a method for preparing a diagnostic agent is provided. The method comprises, preferably consists of, the steps of:
providing the compound of formula 10a and/or 10b; and
formulating the compound with a pharmaceutically acceptable carrier and/or a solvent and, if applicable, further pharmaceutical excipients.

It is preferred that the diagnostic agent is designated for a use within the positron emission tomography.

According to an embodiment of the present invention, the compound of the formula 10a and/or 10b is for use in surgery.

*In vitro* evaluation of the compounds of formula 10a and/or 10b may be performed by measuring the uptake of these compounds in suitable cancer cell lines, such as HCC1937 cells, and comparing with a suitable standard, such as the inactive enantiomer [¹⁸F]LT-674. The vitro evaluation data provided herein clearly suggest that the compounds of formula 10a and/or 10b are also suitable for use *in vivo*, i.e. for use in surgery of a mammal, such as a human, e.g. for following up the progress and/or the result of an operation, such as a cancer surgery.

It will be appreciated that the present invention discloses the provision of any precursors, such as the compound of any of formulas 5d, 5e, 6a, 6b, 7a, 7b, 7c, 7d, 7e, 8a, 8b, 9a, and 9b disclosed herein. Accordingly, preferred embodiments are directed to the provision of one or more of the compounds of any of formulas 5d, 5e, 6a, 6b, 7a, 7b, 7c, 7d, 7e, 8a, 8b, 9a, and 9b as well as the corresponding preparation method.

### BRIEF DESCRIPTION OF THE FIGURES

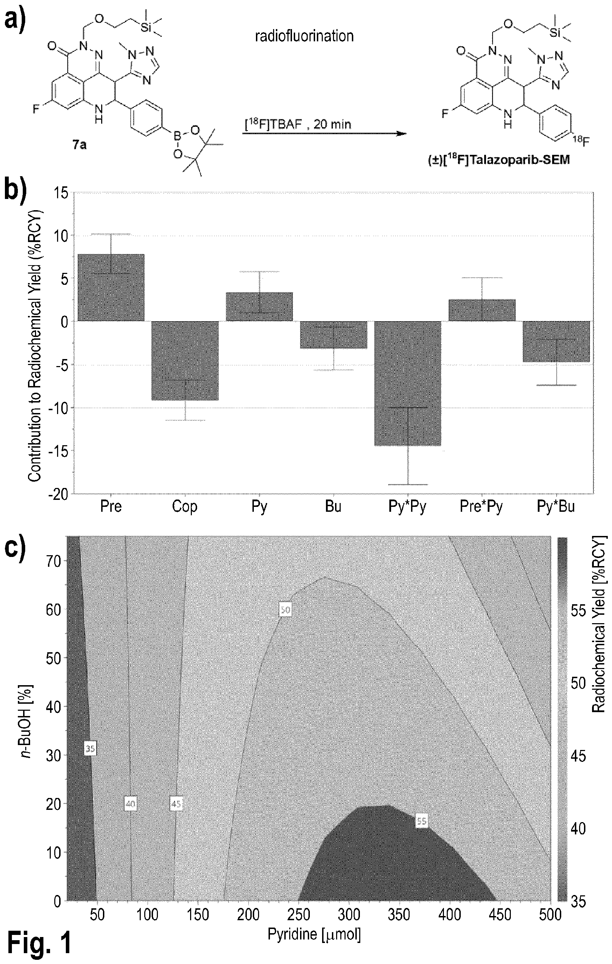

Fig. 1A shows a D-Optimal DoE study that was used to optimize the radiolabeling of the compound 7a. Fig. 1B shows the scaled and centered regression coefficient calculated from the results of D-optimal response surface modeling DoE of the radiosynthesis of [¹⁸F] talazoparib. Large bars represent factors with a large contribution to the response (% radiochemical yield (RCY)). A positive number denotes a positive influence on the response. A negative number indicates a diminishing effect on the response. If a factor's regression coefficient is smaller than the associated error bars, it is probable (at the 95% confidence interval) then that factor is not significant (sample size N=23; goodness of model fit R²=0.946; residual standard deviation RSD=4.412; degrees of freedom D=15; Q2=0.855; Confidence=0.95 - all data calculated in MODDE Go 12 software (Sartorius)). Fig. 1C shows a section of the response surface calculated from the regression model (variables not shown: Cop = 5 µmοl, Pre = 30 µmοl).
Fig. 2 shows another representation of the scaled and centered regression coefficient calculated from the results of D-optimal response surface modelling DoE of the radiosynthesis of [¹⁸F] talazoparib according to Fig. 1B.

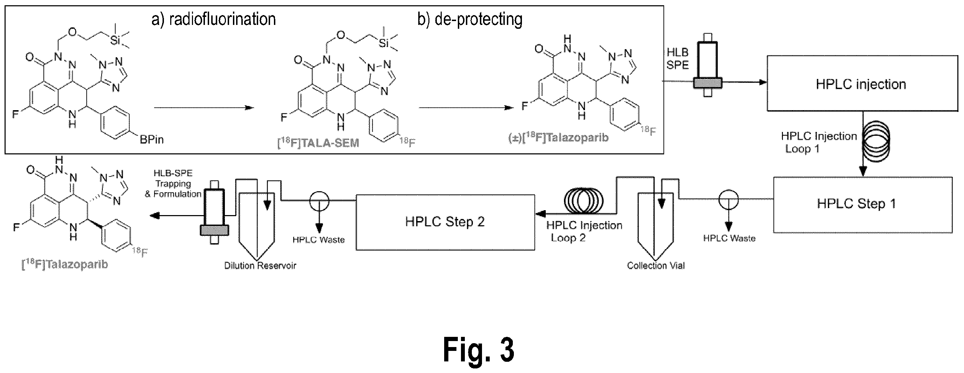

Fig.3 shows the general automated procedure for the radiosynthesis, HLB-SPE clean-up, 2D HPLC purification and chiral resolution, and product concentration and formulation of [¹⁸F] talazoparib. The rectangular box indicates the radiosynthesis step including a) radiofluorination with [¹⁸F]TBAF, Cu(OTf)₂, Py, DMA, n-BuOH, 120°C for 20 min. and b) de-protecting with HCI 6M, 120°C for 10 min. followed by NaOH 6M, in HPLC buffer. The formulation for HPLC injection includes MeCN : water (0.1% TFA) (1:4). HPLC Step 1 includes C18 HPLC MeCN : water (0.1% TFA) (28:72). HPLC Step 2 includes Chiralpak IB-N5 MeCN : water (0.1% TFA) (40:60). The volume of the HPLC injection loops 1 and 2 as well as the collection vial is 5 ml, respectively. The volume of the dilution reservoir is 60 ml.
Fig. 4 shows summary statistics for D-Optimal DoE for CMRF of [¹⁸F] talazoparib. R2 represents the goodness of regression model fit. Q2 represents the goodness of model prediction. "Reproducibility" is calculated from the standard deviation in replicate (centerpoint) experiment results. These statistics represent a valid and predictive regression model.
Fig. 5 shows the connection schematic for the fully automated [¹⁸F] talazoparib radiosynthesis using an Elixys FLEX/CHEM radiosynthesizer coupled to a PURE/FORM formulation and purification module. The cassette activity-in is labelled ¹⁸F from cyclotron. Quaternary methylammonium (QMA) loops are cassette cartridge loop one and two, respectively (cassette one = QMA; cassette two = HLB). The transfer loops of Cassette 1 and Cassette 2 are cassette cartridge loop two, respectively (no cartridge). The arrow connecting Cassette 1 and the dilution reservoir is the cassette activity-out line.
Fig. 6 shows the set-up of the FX N Pro.
Fig. 7 shows the uptake of [¹⁸F] talazoparib (left) and the inactive enantiomer [¹⁸F]LT-674 (right) in HCC1937 cells, blocked with olaparib or talazoparib.
Fig. 8 shows the analytical Chiral HPLC (Chiralpak IB-N5, 5 µm, 150 mm x 4.6 mm) with MeCN: H₂O + TFA (0.1%); 40:60. Fig 8 a) shows a racemic mixture (non-radioactive standard). Fig 8 b) shows talazoparib (non-radioactive std.), and Fig 8 c) shows the inactive enantiomer LT-674 (non-radioactive std.).
Fig. 9 shows a semipreparative Radio-HPLC (IB-N5, 5 µm, 250 mm x 10 mm) MeCN: H₂O + TFA (0.1%); 40:60. The Radio-HPLC with the system of ELIXYS. Peak cuts are affected by the fraction collector.
Fig. 10 shows the analytical Chiral Radio-HPLC (IB-N5, 5 µm, 150 mm x 4.6 mm) with MeCN: H₂O + TFA (0.1%); 40:60. A and B show [¹⁸F] talazoparib (active enantiomer) with a retention time of about 5.8 min, and B and [¹⁸F]LT-674 (inactive enantiomer) with a retention time of about 7.375 min.

### EXAMPLES

### General

All chemicals, reagents, catalysts, and solvents were purchased from either *Sigma Aldrich* (St. Louis, Missouri, USA), *Merck* (Darmstadt, Germany), *abcr GmbH* (Karlsruhe, Germany), *Karl Roth* (Karlsruhe, Germany), and were used without any additional purification unless otherwise stated. QMA, SPE, and SEP-PAK cartridges were obtained from *Waters* (Milford, Massachusetts, USA) unless otherwise stated. Reactions were monitored using thin-layer chromatography (TLC) on 0.20 mm Polygram SIL G/UV₂₅₄ (silica gel 60) TLC plates and were developed with an appropriate running buffer/solvent mixture. Spots were visualized with UV light (254 or 366 nm). Preparative flash chromatography was performed using pre-packed silica gel columns (SNAP KP-Sil or SNAP Ultra (25 µm HP-Sphere), 10 g, 25 g, 50 g, or 100 g, (*Biotage,* Uppsala, Sweden)) on an automated chromatography system (Isolera 4, *Biotage*) which featured a UV detector and fraction collector. Unless otherwise stated, all columns were dry loaded by absorption onto either silica gel or diatomaceous earth packing material (Isolute, *Biotage).* ¹H and ¹³C NMR spectra were obtained at 300 K using an Avance III AV 600 (¹H: 600.13 MHz and ¹³C: 150.61 MHz) spectrometer (*Bruker,* Billerica, Massachusetts, USA). All chemical shifts (δ) are reported in ppm, and all *J* values are reported in Hz. The following abbreviations are used to describe multiplicities: s (singlet), d (doublet), t (triplet), q (quartet), m (multiplet) brs (broad singlet). All compounds were dissolved in chloroform (CDCl₃) unless otherwise stated. All chemical shifts were referenced to residual chloroform (δ_{H} = 7.24 and δ_{C} = 77.00) or DMSO (δ_{H} = 2.50 and δ_{C} = 39.52). Analytical HPLC-MS data was collected using a 1200 series HPLC machine coupled to quadrupole 6120 series MS detector in ESI mode (*Agilent*, Santa Clara, California, USA) under the following conditions: Column: Luna 5µm C18 (2) 100 Å, 50 x 2 mm; Solvent A: H₂O + Formic acid (0.1%); Solvent B: acetonitrile; Gradient: 0-7.60 min (0% - 100% B), 7.60 - 7.80 min (100% B), 7.80 - 8.30 min (100% - 0% B), 8.30 - 12.0 min (0% B).

### Chemical synthesis

1*-Methyl-1H-1,2,4-triazole-5-carbaldehyde (1*). 1-methyl-1H-1,2,4-triazole (10 g, 120 mmol) was dissolved in THF (60 ml) in a dry argon purged two-neck reaction flask fitted with a rubber septum, and the resulting solution was then cooled to 0 °C. A solution of 2M isopropylmagnesium chloride (66 ml, 132 mmol) was then added dropwise through the septum via a syringe over 15 min. The reaction mixture was then allowed to warm slowly to room temperature and stir for 1.5 hours. The reaction vessel was again cooled to 0 °C, after which N,N-dimethyl formamide (DMF) (14 ml, 180.5 mmol) was added dropwise via syringe. The reaction mixture was once more allowed to slowly warm to room temperature and stir overnight. The next morning, the reaction was quenched by the slow addition of HCI (2M) until pH 2, and the resulting mixture was diluted with DCM (100 ml). The phases were separated with a separating funnel, and the aqueous phase was neutralized with saturated aqueous sodium bicarbonate (NaHCO₃) and extracted with DCM (2 x 80 ml). The organic fractions were combined, washed once with brine, dried with magnesium sulfate (MgSO4), and concentrated under reduced pressure to remove the DCM. The product 1 is a volatile oil (≈60 °C) and was thus not purified further. The compound was used, in excess, directly in the next step without further analysis, assuming a synthesis yield of 75%.

*6-Fluoro-3-((1-methyl-1H-1,2,4-triazol-5-yl)methylene)-4-nitroisobenzofuran-1(3H)-one (**2**).* Commercially available 6-fluoro-4-nitroisobenzofuran-1(*3H*)-one (9.8 g, 50 mmol), a solution of **1** in THF (ca. 100 mmol, assuming 75% conversion in the previous step), and triethylamine (21 ml, 150 mmol) were dissolved in dry THF (150 ml) in an argon-filled two-neck reaction flask fitted with a reflux condenser. Acetic anhydride (35 ml) was then added dropwise to the reaction mixture over 3 min, and the resulting mixture was then heated to reflux for 1 hour. The mixture was then removed from the heat and concentrated under reduced pressure to a volume of approximately (10 ml) until a green/yellow precipitate formed. The resulting slurry was then cooled in a freezer to -5 °C, and the solid was collected through vacuum filtration. The resulting cake was washed with cold ethyl acetate, and the residue was then dried under high vacuum for 4 hours to afford **2** as a grey-green solid (7.34 g, 50 %). The analytical data agree with the literature. 1H NMR (600 MHz, DMSO) δ 7.74 (dd, J = 8.7, 2.3 Hz, 1H), 7.56 (dd, J = 6.4, 2.4 Hz, 1H), 7.25 (d, J = 0.6 Hz, 1H), 6.32 (s, 1H), 3.09 (s, 3H).

*Methyl 5-fluoro-2-(2-(1-methyl-1H-1,2,4-triazol-5-yl)acetyl)-3-nitrobenzoate (**3**).* Compound 2 (7.34 g, 25.2 mmol) was suspended in methanol (204 ml), and acetic acid (0.5 ml) was added to the resulting solution. The mixture was then warmed to 50 °C until HPLC-MS confirmed complete consumption of the starting material (4 - 12 hours). The solvents were then removed under high vacuum to afford 3 as a yellow solid in nearly a quantitative yield (8.1 g, 99%). The product was used directly in the next step without further purification. The analytical data agree with the literature. 1H NMR (600 MHz, DMSO) δ 8.52 (dd, J = 8.2, 2.6 Hz, 1H), 8.26 (dd, J = 8.3, 2.6 Hz, 1H), 7.85 (s, 1H), 4.59 (s, 2H), 3.91 (s, 3H), 3.88 (s, 3H). 13C NMR (151 MHz, DMSO) δ 195.48, 163.48, 161.21 (d, J = 252.6 Hz), 150.18, 148.75, 147.10 (d, J = 8.8 Hz), 133.58, 131.24 (d, J = 7.7 Hz), 123.63, 117.09 (d, J = 26.9 Hz), 53.71, 40.55, 35.34.

*8-(4-Bromophenyl)-5-fluoro-9-(l-methyl-lH-1,2,4-triazol-5-yl)-2,7,8,9-tetrahydro-3H-pyrido[4,3,2-de]phthalazin-3-one (**5**)*. Compound **3** (8.1 g, 25.2 mmol) and 4-bromobenzaldehyde (8.9 g, 50.5 mmol) were suspended in THF (50 ml) and MeOH (10 ml). To the resulting mixture was added titanium (III) chloride solution (20% wt solution in HCI (2M), 130 ml, 6 Eq.) in dropwise fashion over 30 min at room temperature. The reaction temperature was maintained between 30 and 50 °C for 2 hours, after which it was quenched by the slow addition of water (260 ml). The reaction mixture was then poured into a separating funnel and extracted with ethyl acetate (4 x 140 ml). The organic fractions were pooled and washed with NaHCO₃ (3 x 60 ml) and NaHSO₃ (3 x 100 ml), dried with sodium sulfate (NaSO₄), and concentrated under reduced pressure to afford a think yellow syrup, which was carefully washed with aliquots of diethyl ether (3 x 10 ml). The resulting yellow syrup was then dried under high vacuum to afford the crude intermediate **4** as a yellow amorphous solid (11.3 g, 98%) that was used in the next step without any further purification.

Intermediate **4** (11.3 g, 24.6 mmol) was dissolved in methanol (30 ml) at room temperature, and to the resulting solution was added hydrazine monohydrate (7.7 ml). The reaction mixture was then left to stir overnight at room temperature. The next morning the resulting white precipitate was collected via vacuum filtration to afford Compound **5** as an off-white solid (4.9g, 45% over two steps). 1H NMR (600 MHz, DMSO) δ = 12.35 (s, 1H), 8.04 (s, 0H), 7.80 (s, 1H), 7.72 (s, 1H), 7.53 (d, J=8.2, 2H), 7.41 (d, J=8.1, 2H), 7.07 (dd, J=8.9, 2.4, 1H), 6.91 (dd, J=11.1, 2.5, 1H), 5.04 - 4.96 (m, 2H), 4.02 (s, 1H), 3.68 (s, 3H).

*8-(4-Bromophenyl)-5-fluoro-9-(1-methyl-1H-1,2,4-triazol-5-yl)-2-((2-(trimethylsilyl)ethoxy)methyl)-2,7,8,9-tetrahydro-3H-pyrido[4,3,2-de]phthalazin-3-one (**6a**) and 8-(4-Bromophenyl)-5-fluoro-9-(1-methyl-1H-1,2,4-triazol-5-yl)-2,7-bis((2-(trimethylsilyl)ethoxy)methyl)-2,7,8,9-tetrahydro-3H-pyrido[4,3,2-de]phthalazin-3-one (**6b**).* Sodium hydride (60% in mineral oil, 2.7 mg, 6.8 mmol) was suspended in DMF (20 ml) in a dry, argon-filled flask, and the resulting solution was then cooled to -15- -18 °C using a bath containing a mixture of acetone and ice. Compound **5** (2 g, 4.5 mmol) was added to the NaH suspension in small portions, and DMF (1 ml) was used to wash any compound off the vessel walls. The deep red/purple solution was allowed to stir for 15 minutes, whereupon a solution of (trimethylsilyl)ethoxymethyl chloride (1.13 g, 6.8 mmol) in DMF (4 ml) was added dropwise through a rubber septum over 5 minutes. Throughout the addition, the solution turned clear and light orange and was allowed to stir for a further 20 min before being quenched with sat. Aq. NH₄Cl (20 ml). The reaction mixture was diluted with water (250 ml) and transferred to a separating funnel, where it was extracted with ethyl acetate (3 x 50 ml). The organic fractions were pooled, dried with MgSO₄ and evaporated to dryness. The crude product residue was then purified using flash chromatography (25-75% EtOAc in hexanes) to afford compounds **6a** and **6b** as a white crystalline solid and thick colorless oil (**6a:** 1.447 g, 56 %; **6b:** not determined). Despite the presence of several minor impurities both compounds were used in the next step without further purification.

*5-fluoro-9-(1-methyl-1H-1,2,4-triazol-5-yl)-8-(4-(4,4,5,5-tetramethyl-1,3,2-dioxaborolan-2-yl)phenyl)-2-((2-(trimethylsilyl)ethoxy)methyl)-2,7,8, 9-tetrahydro-3H-pyrido[4,3,2-de]phthalazin-3-one (**7a**).* Compound **6a** (1.448 g, 2.534 mmol), bis(pinacolato)diboron (1.415 g, 5.574 mmol), and potassium acetate (746 mg, 7.602 mmol) were suspended in DMF (22 ml) in a dry argon filled reaction flask fitted with a reflux condenser. [1,1'-Bis(diphenylphosphino)ferrocene]dichloropalladium(II) (Pd(dppf)Cl₂, 371 mg, 0.507 mmol, 20 mol%) was added to reaction mixture and DMF (3 ml) was used to wash the catalyst of the vessel walls. The reaction flask was purged with argon, and the reaction was heated to 90 °C for 4 hours until HPLC-MS showed conversion of the starting material into the desired product. The reaction mixture was diluted with ethyl acetate (80 ml) and passed through a tightly packed plug of Celite^{®} under vacuum. The filtrate was collected and transferred to a separating funnel where it was washed with water (200 ml). The aqueous layer was further extracted with ethyl acetate (3 x 80 ml), after which the organic fractions were pooled, washed with brine, dried with MgS04, and concentrated under reduced pressure to afford the desired product racemate (**7a**) as an off-white solid (1.391 g, 89%).

For radiochemical experiments, the product (515 mg) was further purified by recrystallization. The product was dissolved completely in a hot mixture of acetone and acetonitrile (1:1), and deionized water was then added dropwise until the solution became turbid. The solution was again lightly heated until the solution was nearly transparent, and it was then allowed to slowly cool to the ambient temperature, after which the recrystallization vessel was placed in a refrigerator at 4 °C and allowed to sit overnight. The next day the pure product was collected via vacuum filtration to afford a pure racemic mixture of **7a** as a white crystalline solid (361 mg, 70% recovery). H1 and C13, HPLC: HRMS 1H NMR (600 MHz, CDCl3) δ 7.85 (s, 1H), 7.73 (d, J = 7.8 Hz, 2H), 7.41 (dd, J = 9.7, 2.3 Hz, 1H), 7.36 (d, J = 7.8 Hz, 2H), 6.75 (dd, J = 9.7, 2.3 Hz, 1H), 5.43 (d, J = 9.9 Hz, 1H), 5.26 - 5.18 (m, 2H), 5.02 (s, 1H), 4.52 (d, J = 11.0 Hz, 1H), 3.57 (s, 3H), 2.44 - 1.60 (m, 4H), 1.33 (s, 12H), 0.92 - 0.84 (m, 2H), 0.0 (s, 9H).

The present inventors adapted the synthesis of the precursor from the synthetic route employed by Wang *et al.* for the synthesis of talazoparib (Wang, B.; Chu, D.; Feng, Y.; Shen, Y.; Aoyagi-Scharber, M.; Post, L. E., Discovery and Characterization of (8S,9R)-5-Fluoro-8-(4-fluorophenyl)-9-(1-methyl-1H-1,2,4-triazol-5-yl)-2,7,8,9-te trahydro-3H-pyrido[4,3,2-de]phthalazin-3-one (BMN 673, talazoparib), a Novel, Highly Potent, and Orally Efficacious Poly(ADP-ribose) Polymerase-1/2 Inhibitor, as an Anticancer Agent. J Med Chem 2016, 59 (1), 335-57). It will be appreciated, however, that also other synthetic routes may be used as starting points for the provision of the precursor of formula 5c.

### Precursor synthesis

The precursor of the formula 5c was synthesized as mentioned hereinafter. As may be derived from scheme 1, 1-methyl-1H-1,2,4-triazole was formylated to 1 via the published procedure (W. Hungate; Tae-Seong Kim; Richard T. Lewis; Longbin Liu; Julia Lohman; Mark H. Norman; Michelle Potashman; Aaron C. Siegmund; Stephanie K. Springer; Markian Stec; Ning Xi; Kevin Yang, B. K. A. D. B. S. B. C. M. B. S. B. A. B. D. C. D. D. A. J.-C. H. S. H. R. FUSED HETEROCYCLIC DERIVATIVES AND METHODS OF USE. 2009). **1** was then condensed with commercially available 6-fluoro-4-nitroisobenzofuran-1(3H)-one in THF in the presence of NEt₃ and Ac₂O to afford **2** in a 53% yield. The lactone **2** was opened to give the keto-ester derivative **3** by warming **2** in methanol with a catalytic amount of acetic acid. In the original synthesis, **3** was then reacted with 4-fluorobenzaldehyde in THF and methanol via reductive cyclization driven by TiCl₃ to afford **4b,** which was cyclized with hydrazine monohydrate to afford (±)talazoparib (**5b**), which was to be used as reference compound. Reacting **3** with 4-bromobenzaldehyde via the same two-step procedure afforded the derivatizable bromide **5a.**

A protecting group strategy using the trimethylsilylethoxymethyl ether (SEM) protecting group was employed to the compound **5a,** which was protected with trimethylsilylethoxymethyl chloride (SEMCI) using sodium hydride in DMF at 15 °C. These conditions afforded both the mono- (**6a**) and di-SEM (**6b**) protected bromide derivatives. It is clear, however, that the protecting group strategy is not limited to the disclosed compounds.

Compounds **6a** and **6b** were then further derivatized to yield several possible precursors for radiolabeling (Scheme 2). The protection of **5a** favored the formation **6a** over **6b** (ca. 3:1), even under conditions designed to drive the formation of the di-SEM protected derivative. Compounds **6a** and **6b** were converted to their corresponding boronic acid pinacol esters under Miyaura borylation conditions in DMF with KOAc, bis(pinacolato)diboron, and Pd(dppf)Cl₂ as the catalyst (Scheme 2). This afforded the compounds **7a** and **7b** in good to excellent yields. **7a** was purified through recrystallization a shelf-stable and convenient-to-handle white crystalline solid. **7b** however, was purified through chromatography and was much harder to obtain in the high purities required for reliable radiochemistry. Despite being of high chromatographic purity (HPLC-MS), samples of **7b** displayed a complex NMR spectrum, indicative of the presence of multiple conformation isomers. CMRF chemistry has also been well established with aryl stannanes precursors and he present inventors have investigated this variation of the CMRF reaction extensively in our previous work. Thus, the analogous stannylation of **6a** was carried with bis tributyltin to afford the stannyl precursor **7c** as an amorphous glass.

Optimal chiral HPLC conditions were screened using racemic (±)talazoparib in combination with commercially obtained authentic samples of talazoparib and LT-674. CHIRALPAK IA-U, IB-U, IC-U and IG-U UHPLC columns were tested for their abilities to resolve the two enantiomers under reverse-phase conditions (Water (0.1% TFA):Acetonitrile). CHIRALPAK IB packing material was found to provide a good enantiomeric resolution at relatively low retention time. CHIRALPAK IB-N5 (5 µm) columns under reverse-phase conditions would thus be used for both semipreparative enantiomeric resolution of [¹⁸F] talazoparib and [¹⁸F]LT-674 and for chiral quality control (QC) analysis.

### Automated Radiochemistry

With the precursors **7a-c** in hand, the inventors set out to evaluate which precursor would provide the best radiosynthesis performance, while still being relatively easy to produce, shelf-stable, and convenient to work with. A series of pilot experiments, carried out using each precursor under a small set of arbitrarily chosen unoptimized reaction conditions, determined that compounds **7b** and **7c** displayed only marginally better reaction performance (data not shown) than **7a** (8-15% RCY using unoptimized reaction conditions). As **7a** proved easier to synthesize, purify, and characterize, as well as being shelf-stable, it was chosen as the precursor for further development and optimization.

All radiochemical reactions and experiments were carried out behind appropriate shielding in accordance with the rules and guidelines laid out in the German Strahlenschutzverordnung (StrlSchV).

The inventors have previously explored the use of DoE to solve complex radiochemical optimization problems and have developed a ¹⁸F processing method and workflow that is compatible with small scale DoE radiochemical experiments shown in DoE Studies hereinafter. The inventors applied this workflow to a computer-generated D-optimal DoE study designed investigate the effects of precursor load (*Pre,* 5-30 µmοl), the copper-mediator load (*Cop,* 5-40 µmοl), the pyridine load (*Py*, 20-500 µmοl), and the % of n-BuOH co-solvent (*Bu,* 0-75%) in DMA, on reaction performance (Figs. 1A and 1B). The study consisted of 24 experiments (including centerpoints) and was conducted over 4 days (5 runs/day) using [¹⁸F]fluoride from cyclotron target washes (table 1). Each run was carried out for 20 minutes at 120 °C before evaluation by radioTLC. Analysis of the data revealed the presence of one outlier (table 1, exp 21) which was excluded from the final regression model. The remaining data (n = 23) was used to fit a regression model with R² = 0.946 (goodness of model fit) and Q² = 0.855 (goodness of model prediction) suggesting the model to be valid and predictive.

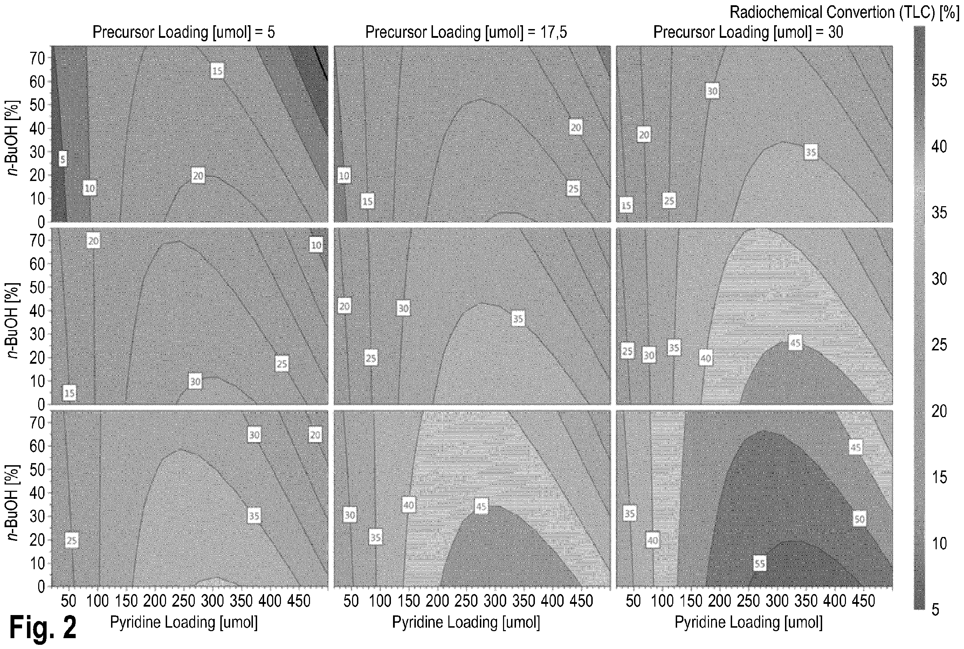

The use of larger amounts of precursor was found to have a positive effect on reaction performance, while smaller amounts of the copper mediator (Cu(OTf)₂) were beneficial. A small amount of n-BuOH (5-10%) was also found to provide a small increase to reaction performance. It is though that n-BuOH increases the rate of the CMRF transmetalation step. Factor interactions (where the setting of factor effects the behavior of another) were found between pyridine and the precursor amount as well as pyridine and n-BuOH. The effect of pyridine on reaction performance was found to possess strong quadratic (py², curved) behavior. Optimal reaction conditions of 30 µmol **7a,** 300 µmol pyridine, and 5 µmol Cu(OTf)₂ in DMA with 10% n-BuOH, were chosen from the response surface plot generated from the regression model (**Fehler! Verweisquelle konnte nicht gefunden werden.**C; see Figure 2 for a full 4D response surface plot.) These conditions were validated manually using full batches of processed [¹⁸F]TBAF and were able to produce protected (±)[¹⁸F] talazoparib-SEM with 58 ± 7.4 %RCY. These results align with DoE model and afford the product in yields acceptable for automation and enantiomeric resolution. Quantitative deprotection to (±)[¹⁸F] talazoparib (>95% conversion according to HPLC) was achieved with 6 M HCI at 100 °C for 15 min.

The optimized CMRF radiosynthesis was translated onto two separate synthesis platforms: An Elixys Flex/Chem automated radiosynthesizer coupled to a Pure/Form purification and formulation module (Sofie biosciences, USA), and a FX_{N} Pro (GE, Sweden). The Elixys Flex/Chem platform is a cassette-based system (up to 3) and is able to perform complex multi-reactor radiosynthesis, while the Pure/Form module is equipped with two semipreparative HPLC injection loops (5 ml) and up to 3 selectable HPLC columns. This setup would therefore allow for the sequential HPLC based purification and enantiomeric resolution required for the synthesis of [¹⁸F] talazoparib. The Tracerlab FX N Pro is a fixed fluid path system with a single HPLC injection loop and column; therefore, the purified product racemate must be transferred to a secondary external HPLC system (in this case an Elixys Pure/Form) for enantiomeric resolution. While the exact technical details differ between the two synthesis modules (the automated radiosynthesis using both systems are described in detail in the SI), the automated synthesis of [¹⁸F] talazoparib follows the same general procedure irrespective of which module is used (.

### Fig).

The DoE optimized reaction mixture was added to the processed dry [¹⁸F]TBAF and reacted at 120 °C for 20 minutes. This was followed by the removal of the SEM protecting group with 6M HCl 120 °C for 10 min. The pH of the reaction mixture was then adjusted to pH 5-7 with the addition of NaOH (2M). The reaction mixture was then diluted and passed over an HLB SPE cartridge (Waters), trapping the product (±)[¹⁸F] talazoparib and removing residual salts and unreacted [¹⁸F]fluoride. The product is then eluted from the HLB cartridge with acetonitrile (1 ml) and reformulated with HPLC buffer for purification in the first HPLC step (C-18 reverse-phase). The product HPLC peak is isolated (< 5 mL) and this solution is then transferred to a second HPLC injection loop for enantiomeric resolution using a semipreparative CHIRALPAK IB-N5 (5µm, 10 x 250 mm) column operating under reverse-phase conditions. The solvents and columns used for the chiral separation HPLC step were carefully chosen to be directly compatible with the conditions of the first HPLC step. The product enantiomer is then isolated, diluted, and trapped on a second HLB cartridge. The product [¹⁸F] talazoparib is then eluted from the cartridge and formulated for injection with ethanol (5%) and phosphate buffered saline (PBS). This procedure was able to produce enantiomerically enriched (>99% ee) [¹⁸F] talazoparib with 12 ± 1.5% RCY (activity yield = 4-6% over 120 min); molar activity = 45-176 GBq/µmol. Product identity, chemical and radiochemical purity, and molar activity was determined using a CHIRALPAK IB-N5 (5 µm, 4.6 X 150 mm) analytical HPLC against the commercially available non-radioactive standard compound.

*¹⁸F Processing.* ¹⁸F processing may be carried out by using known procedures. For instance, [¹⁸F]Fluoride, delivered from the cyclotron in an aqueous solution, was passed over a preconditioned QMA-OTf cartridge. (The QMA cartridge was conditioned using 10 ml KOTf solution in water (90 mg/ml), followed by air (10 ml), followed by water (10 ml), followed by air (10 ml)). The [¹⁸O]H₂O (or cyclotron target wash water) was collected in a separate vial for recycling, and a stream of argon was used to blow any residual water off the QMA cartridge. Once "air dry," the [¹⁸F]fluoride was eluted from the QMA as [¹⁸F]TBAF, using a solution of TBAOTf (10 mg) in methanol (1 ml). The resulting solution of [¹⁸F]TBAF in methanol was then either distributed into aliquots for DoE optimization experiments or used as a full batch for tracer productions. The [¹⁸F]TBAF was added into a reactor vessel, and the methanol was removed by evaporation under a stream of argon gas to afford dry [¹⁸F]TBAF without the need for extended azeotropic drying.

To dry [¹⁸F]TBAF is a single used glass reactor vessel was added a premade solution containing the required quantities of the precursor for radiolabeling (7a-c), Cu(OTf)₂, pyridine, DMA, and n-BuOH (total reaction volume of 700 µl). The reaction mixture was then stirred for at 120 °C for 10 minutes before being quenched with HCI (0.25 M, 700 µl) or reacted further with 6M HCI. Reaction performance was evaluated using radioTLC, which was read out using a Cyclone Plus storage phosphor imaging system (*PerkinElmer*, Waltham, Massachusetts, USA). Compound identity was confirmed by analytical radioHPLC against the non-radioactive standard compound.

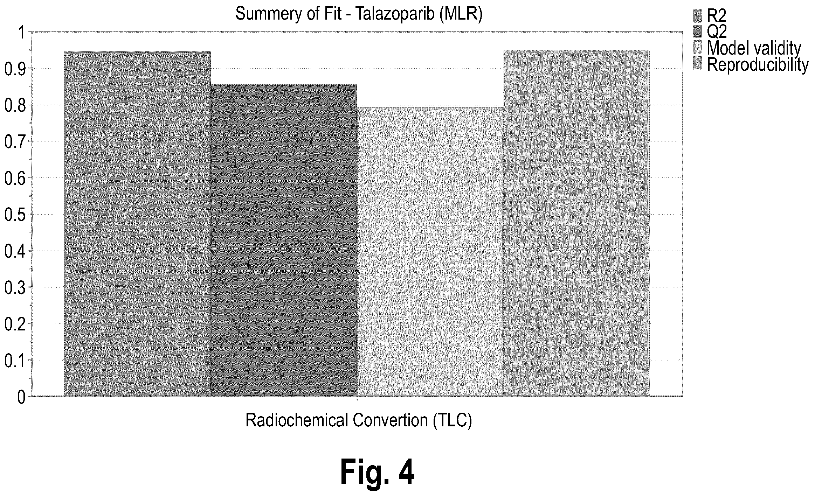

Fig. 3 shows the general automated procedure for the radiosynthesis. From Fig. 4 the summary statistics for D-Optimal DoE for CMRF of [¹⁸F] talazoparib are derivable.

### DoE Studies

A computer-generated D-optimal DoE (design of experiments) study was designed and analyzed using *MODDE Go 12* (Sartorius, Germany). The D-optimal DoE study was performed to maximize the radiochemical yield of the CMRF step of the [¹⁸F] talazoparib radiosynthesis across four experimental factors: The precursor load (*Pre,* 5-30 µmol), the copper-mediator load (*Cop,* 5-40 µmοl), the pyridine load (*Py*, 20-500 µmοl), and the % of *n*-BuOH co-solvent (*Bu,* 0-75%). The resulting worksheet table (table 1) was used to calculate the required reactants, reagents, and solvents for each experiment, and the study was performed over 4 days using four cyclotron target washes. All DoE experiments were carried out in a randomized order. All reactions (tests and tracer productions) were carried out using a racemic mixture of protected (**7a**) precursor. In all cases, [¹⁸F]fluoride was processed into [¹⁸F]TBAF via the general procedure described above. The response (Y) was the %RCY of the labeling reaction and was measured by radio TLC (100% ethyl acetate development solvent). Selected runs were analyzed via radio HPLC against a non-radioactive standard to confirm compound identity.

**Table 1: The DoE Worksheet table. Experiments were performed in randomized order and are listed in the order in which they were carried out.**

| Exp No | Run Order | Precursor Loading (µmol) | Precurs or (mg) | Cu(OTf)₂ (µmol) | Cu(OTf)² (mg) | Pyridine Loading (µmol) | Pyridine (mg) | Pyridine (µl) | n-BuOH % | n-BuOH (µl) | RXN Vol (µl) | RCC (%) |
|---|---|---|---|---|---|---|---|---|---|---|---|---|
| 4 | 1 | 5 | 3 | 40 | 14 | 500 | 39.55 | 40.4 | 0 | 0 | 500 | 10.4 |
| 18 | 2 | 13.3333 | 8 | 5 | 2 | 500 | 39.55 | 40.4 | 75 | 375 | 500 | 13.7 |
| 16 | 3 | 30 | 19 | 28.3333 | 10 | 500 | 39.55 | 40.4 | 75 | 375 | 500 | 25.6 |
| 21 | 4 | 17.5 | 11 | 22.5 | 8 | 20 | 1.582 | 1.6 | 37.5 | 187.5 | 500 | 36.3 |
| 13 | 5 | 30 | 19 | 5 | 2 | 180 | 14.238 | 14.6 | 75 | 375 | 500 | 47.1 |
| 9 | 6 | 5 | 3 | 5 | 2 | 500 | 39.55 | 40.4 | 25 | 125 | 500 | 26.4 |
| 20 | 7 | 17.5 | 11 | 40 | 14 | 260 | 20.566 | 21.0 | 37.5 | 187.5 | 500 | 27.2 |
| 14 | 8 | 30 | 19 | 40 | 14 | 500 | 39.55 | 40.4 | 25 | 125 | 500 | 26 |
| 1 | 9 | 5 | 3 | 5 | 2 | 20 | 1.582 | 1.6 | 0 | 0 | 500 | 15.9 |
| 17 | 10 | 21.6667 | 13 | 5 | 2 | 500 | 39.55 | 40.4 | 0 | 0 | 500 | 48.3 |
| 10 | 11 | 30 | 19 | 5 | 2 | 20 | 1.582 | 1.6 | 25 | 125 | 500 | 35.7 |
| 5 | 12 | 5 | 3 | 5 | 2 | 20 | 1.582 | 1.6 | 75 | 375 | 500 | 27.5 |
| 19 | 13 | 5 | 3 | 22.5 | 8 | 260 | 20.566 | 21.0 | 37.5 | 187.5 | 500 | 22.3 |
| 3 | 14 | 30 | 19 | 40 | 14 | 20 | 1.582 | 1.6 | 0 | 0 | 500 | 11 |
| 24 | 15 | 17.5 | 11 | 22.5 | 8 | 260 | 20.566 | 21.0 | 37.5 | 187.5 | 500 | 32.7 |
| 23 | 16 | 17.5 | 11 | 22.5 | 8 | 260 | 20.566 | 21.0 | 37.5 | 187.5 | 500 | 38.9 |
| 7 | 17 | 30 | 19 | 40 | 14 | 20 | 1.582 | 1.6 | 75 | 375 | 500 | 10.9 |
| 22 | 18 | 17.5 | 11 | 22.5 | 8 | 260 | 20.566 | 21.0 | 37.5 | 187.5 | 500 | 38.7 |
| 15 | 19 | 30 | 19 | 16.6667 | 6 | 500 | 39.55 | 40.4 | 0 | 0 | 500 | 45.5 |
| 11 | 20 | 30 | 19 | 5 | 2 | 500 | 39.55 | 40.4 | 50 | 250 | 500 | 40.5 |
| 12 | 21 | 30 | 19 | 5 | 2 | 340 | 26.894 | 27.5 | 0 | 0 | 500 | 57.8 |
| 2 | 22 | 5 | 3 | 40 | 14 | 20 | 1.582 | 1.6 | 0 | 0 | 500 | 3.7 |
| 8 | 23 | 5 | 3 | 40 | 14 | 500 | 39.55 | 40.4 | 75 | 375 | 500 | 4.3 |
| 6 | 24 | 5 | 3 | 40 | 14 | 20 | 1.582 | 1.6 | 75 | 375 | 500 | 3.3 |

The detailed automated radiosynthesis of [¹⁸F] talazoparib, using both the Elixys (Sofie bioscience, USA) and FX N pro (GE, Sweden) systems is described in detail in hereinafter.

The general procedure is as follows: [¹⁸F]Fluoride in [¹⁸O]water was delivered into the module directly from the cyclotron where it was trapped on a QMA cartridge (OTf-form). The [¹⁸O]water was collected for recycling. The ¹⁸F was then eluted with a solution of tetrabutylammonium triflate (TBAOTf) in methanol (1 ml), which was evaporated to dryness under a stream of argon to afford base-free [¹⁸F]TBAF. The DoE optimized reaction mixture, consisting of **7a** (19 mg, 30 µmοl), Cu(OTf)₂ (3 mg, 5 µmοl), and pyridine (24 µl, 300 µmol) in 700 µl DMA with n-BuOH (10%), was then added to the [¹⁸F]TBAF reacted at 120 °C for 20 min. After cooling the reactor vessel to ambient temperature HCI (6M, 700 µl) was added to the mixture and reacted at 120 °C for 10 min. The reaction vessel was again cooled, and the reaction was quenched with the addition of NaOH (2 M, 2.1 ml). The mixture was diluted with water (<15% organic solvents) and passed over an HLB SPE cartridge, trapping the product. The product was then eluted into a 5 ml vessel with acetonitrile (1 ml) and HLPC buffer (4 ml) and this solution was transferred to the first HPLC injection loop (5ml). The product racemate was then isolated from the reaction mixture using reverse-phase HPLC (C-18 Luna (10 µm, 10 x 250 mm)) via an isocratic method using water (0.1% TFA):acetonitrile (72:28). The radioactive fraction was collected in seal v-vial that was subsequently pressurized to load the solution onto a second HPLC injection loop. The purified racemic product was then injected on to a semipreparative CHIRALPAK IB-N5 (5 µm, 10 x 250 mm) HPLC column for enantiomeric resolution. The radioactive fraction corresponding to [¹⁸F] talazoparib was collected in a dilution reservoir, the contents of which was subsequently passed over an HLB cartridge. The product [¹⁸F] talazoparib was eluted from the cartridge with ethanol (0.5 ml) and reconstituted with PBS (4.5 ml).

*Quality control.* Product identity was confirmed with analytical CHIRALPAK IB-N5 (5 µm, 4.6 x 150 mm) HPLC against a commercially acquired non-radioactive standard samples of talazoparib and its biologically inactive enantiomer LT-674. The molar activity of the radiotracer was calculated from the analytical HPLC UV signal using a calibration curve generated from the serial dilution of a standard sample of talazoparib.

The automated radiosynthesis of [¹⁸F] talazoparib may feature the following process steps: 1) Radiosynthesis of (±) [¹⁸F] talazoparib. 2) Semi-preparative HPLC of (±)[¹⁸F] talazoparib. 3) Enantiomeric separation of [¹⁸F] talazoparib and [¹⁸F]LT-674 via Semi-preparative Chiral-PAK HPLC . 4) Tracer concentration and formulation. It will be clear, however, that this segregation is for better understanding and may be employed in a different manner.

All four steps could be performed using an Elixys FLEX/CHEM synthesizer coupled to a PURE/FORM concentration and formulation module. Alternatively, steps 1) and 2) could be performed using a FX N Pro (GE), after which the compound was transferred to an external purification and Formulation module for steps 3) and 4).

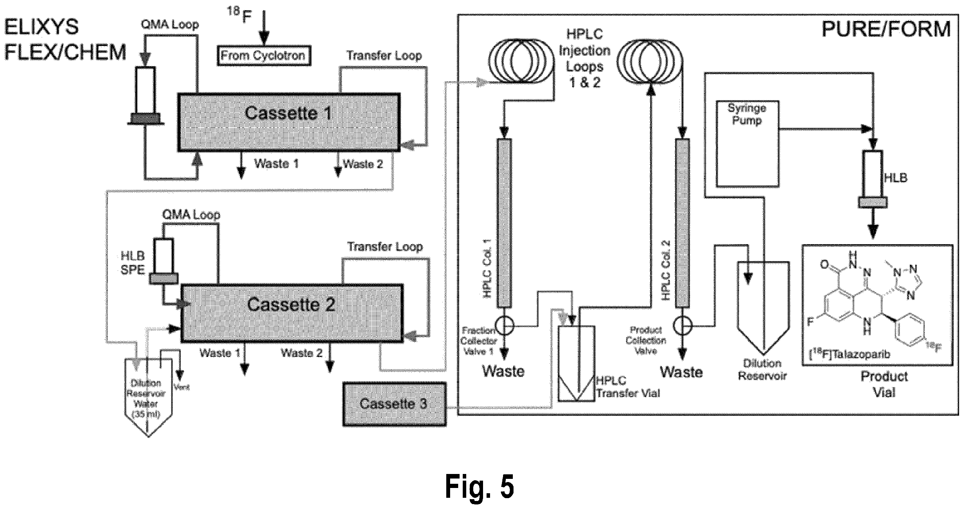

The radiosynthesis of [¹⁸F] talazoparib was performed using an Elixys Flex chem with three Elixys cassettes and three 5 ml v-vial reactor vessels. Each cassette was loaded with prefilled reagent vials in accordance with the table 2. A pear-shaped distillation flask was used as a dilution reservoir and was situated between cassettes 1 and 2. The cassettes were set up and connected as shown in Fig. 5. Cassette 1 is for radiosynthesis and deprotection. Cassette 2 is for formulation for HPLC injection. HPLC Col. 1: C-18, solvent: water (0.1% TFA): MeCN; (solvent ratio 72:78). HPLC Col. 2: IB-N5, Water (0.1% TFA): MeCN; (solvent ratio 60:40). The syringe pump is for 1. water, 2. ethanol, 3. PBS. The PURE/FORM syringe pump was primed before each synthesis.

**Table 2: Synthesis reagents, cartridges, and eluents, and their corresponding cassette positions.**

| **Cassette and Reagent Position** | **Reagent** |
|---|---|
| Cassette 1; Position 1: QMA Eluent | TBAOTf in Methanol (10 mg/ml) 1ml |
| Cassette 1; Position 2: Reaction Mixture | **7a** (19 mg); **Cu(OTf)₂** (3 mg); **Pyridine** (24 µl); **DMA** (606 µl); ***n*** - **BuOH** (70 µl |
| Cassette 1; Position 3 | HCl 6M (700 µl |
| Cassette 1; Position 4 | Ammonium Formate Buffer (25 mM, 1.5 ml) + NaOH (6M, 300 µl) |
| Cassette 1; Position 5 | Ammonium Formate Buffer (25 mM, 1.5 ml) + NaOH (6M, 300 µl) |
| Cassette 1; Position 6 | Acetonitrile(1 ml) |
| Cassette 2; Position 1 | Water (0.1% TFA) (2 ml) |
| Cassette 2; Position 2 | Water (0.1% TFA) (2 ml) |
| Dilution Reservior 2 (PURE/FORM) | Water (60 ml) |
| Cassette 1; Cartridge Loop 1 (BLUE) | QMA (KOTf Preconditioned) |
| Cassette 1; Cartridge Loop 2 (RED) | HLB (Conditioned EtOH (2 ml), water (2 ml)) |
| PURE/FORM SPE Loop | HLB (Conditioned EtOH (2 ml), water (2 ml)) |
| HPLC Eluent A | Water (0.1% TFA) |
| HPLC Eluent B | Acetonitrile |
| HPLC Column 1 | C-18 Luna (10 µm, 10 mm x 250 mm) |
| HPLC Column 2 | CHIRALPAK IB-N5 (5 µm, 10 mm x 250 mm) |
| PURE/FORM Syringe Pump "water" | Water (40 ml) |
| PURE/FORM Syringe Pump "Ethanol" | Ethanol (20 ml) |
| PURE/FORM Syringe Pump "Saline" | PBS (40 ml) |

The Elixys sequence for the radiosynthesis of [¹⁸F] talazoparib (listed as Elixys unit operations) is as follows: Step 1: Trap Isotope (¹⁸F delivered directly from the cyclotron, over the QMA cartridge in Position A). Step 2: Elute Isotope (Cassette 1: Position 1: QMA Eluent). Step 3: Evaporate (90 °C, 4-7 minutes or until no liquid is observed through reactor camera). Step 4: Add Reagent (Cassette 1: Position 2: Reaction Mixture). Step 5: Move Reactor (Blow 20 ml of air into the reactor vessel with a syringe fitted with a long needle). Step 6: React (120 °C, 20 min)Step 7: Add Reagent (Cassette 1: Position 3: HCI (700 µl)). Step 8: React (100 °C, 15 min). Step 9: Add Reagent (Cassette 1: Position 4: water (2 ml)). Step 10: Transfer (Out to collection vial (dilution reservoir 1)). Step 11: Add Reagent (Cassette 1: Position 5 water, (3 ml)). Step 12: Transfer (Out to collection vial (dilution reservoir 1)). Prompt 13: Remove the needle from cassette 1 and the vent needle from the dilution reservoir. Step 14: Trap Isotope (From external vial, over the HLB (*Waters*) cartridge in position B, 8 minutes). Step 15: Elute Isotope (Cassette 2: Position 1: acetonitrile (1 ml)). Step 16 and 17: Add Reagent (Cassette 2: Positions 2 & 3: HPLC Aqueous phase (4 ml)). Step 18: Transfer (Cassette 2 to PURE/FORM Loop 1 (manual injection)). When the transfer is complete (fluid detector reads "No Fluid"), trigger HPLC injection manually. Step 19: Semiprep HPLC 1. Column: C-18 Luna (10 µm, 10 x 250 mm). HPLC Eluent (Isocratic): 28 % Acetonitrile; 72 % Water (w/ 0.1% TFA); 6 ml/min. The product radio peak elutes at approximately 10:30-13 min and is cut into a sealed v-vial (10 ml) fitted with a vent, a connection to cassette 3, and long needle pushed to bottom of the vial for transfer to HPLC loop 2. Prompt 20: After the radioactive fraction has been collected, remove the vent needle form the HPLC transfer vial. Step 21: Transfer (Cassette 3 (only provides gas pressure) to PURE/FORM Loop 1 (manual injection)). Step 22: Semiprep HPLC 2. Column: CHIRALPAK IB-N5 (5 µm, 10 x 250 mm) fitted with CHIRALPAK IB-N5 (5 µm, 10 x 20mm) column guard. HPLC Eluent (Isocratic): 40 % Acetonitrile; 60 % Water (w/ 0.1% TFA); 5 ml/min. The product enantiomer peak elutes at approximately 9-10 min (see attached HPLC Trace) and is cut into the PURE/FORM Dilution reservoir (Containing 60 ml water) for SPE reformulation. The Inactive enantiomer elutes at 14:30-15:30 min and can be isolated using the Elixys fraction collector if need be. Step 23: Formulation. The dilution reservoir contents are passed over an HLB cartridge, trapping the purified [¹⁸F]olaparib. Ethanol (0.5 ml) is then used to elute the radiotracer into a product vial. PBS (4.5 ml) is then used to reconstitute the tracer in the product vial.

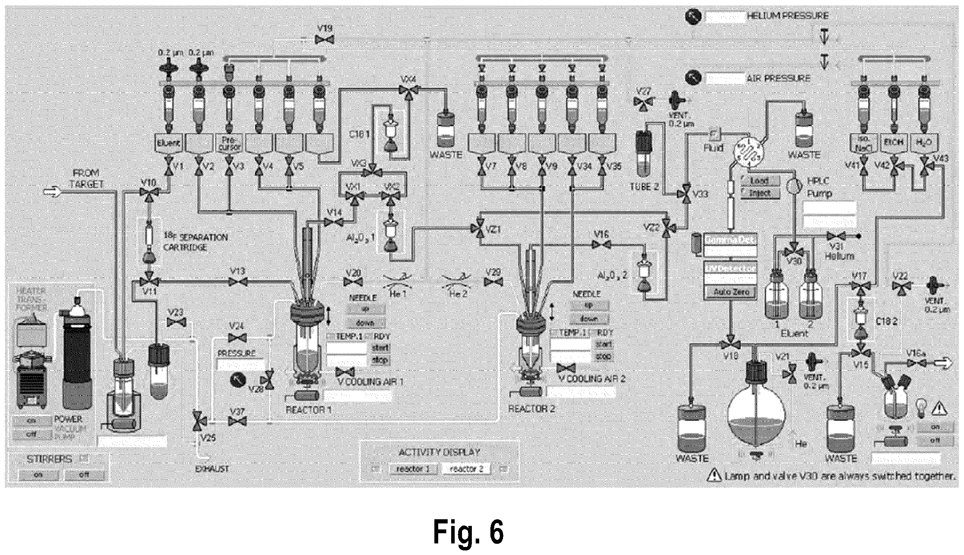

Semi-automated Radiosynthesis of [¹⁸F] talazoparib is performed using a GE FX N Pro Radiosynthesis System and Elixys PURE/FORM module. The FX N Pro was set up as depicted in Fig. 6. Reagent vials were filled as described in the table 3 below.

**Table 3: Synthesis reagents, cartridges, and eluents and their corresponding positions.**

| Reagent Position | Reagent |
|---|---|
| Vial 1: QMA Eluent | TBAOTf in Methanol (10 mg/ml) 1 ml |
| Vial 2: Reaction Mixture | **OLA-Bpin** (7 mg); **[Cu(OTf)₂(Impy)₄]** (18 mg); DMI (700 µl) |
| Vial 3: | TFA (700 µl) |
| Vial 4: | Ammonium Formate Buffer (25 mM, 12.5 ml) |
| Vial 5: | Methanol Wash (0.5 ml) |
| Vial 6: | Acetonitrile (1 ml) |
| Tube 2: For HPLC injection | Ammonium Formate Buffer (25 mM, 3.5 ml) |
| Vial 12: Formulation | PBS (4.5 ml) |
| Vial 13: Formulation | EtOH (0.5 ml) |
| Vial 14: Formulation | Water (4 ml) |
| Dilution Reservior (Crystal Ball) | Water (60 ml) |
| QMA Cartridge Slot: QMA | QMA (KOTf Preconditioned) |
| C-18 Cartridge Slot 1: HLB | HLB (Conditioned EtOH (2 ml), water (2 ml)) |
| C-18 Cartridge Slot 2: HLB | HLB (Conditioned EtOH (2 ml), water (2 ml)) |
| HPLC Eluent A: (For purification) | Ammonium Formate Buffer (25 mM) |
| HPLC Eluent B: (For column flush) | Acetonitrile |
| HPLC Column | C-18 Luna (10 µm, 10 mm x 250 mm) |

The synthesis protocol is shown hereinafter. At the end of bombardment (EOB), ¹⁸F was delivered from the cyclotron into a delivery vial contained within the FX N pro. The contents of the vial were then drawn over the QMA cartridge by vacuum, and the [¹⁸O]water was collected in a separate waste vial for recycling. The ¹⁸F was then eluted from the QMA with TBAOTf (10 mg) in methanol (1 ml) into the reactor. The walls of the reaction vessel were then washed with methanol (0.5 ml) from vial 5, and the methanol was removed by evaporation at 90 °C for 5 minutes under vacuum and a stream of helium. The reaction mixture was drawn into the reaction vessel with vacuum from vial 2. As vial 2 was open to the air, air was thus also drawn into the reaction vessel over 1 minute. The reactor was sealed and heated to 120 °C for 20 minutes, after which it was cooled to 45 °C, and the contents of vial 3 (TFA, 700 µl) were pushed into the reactor using helium carrier gas. The reactor was then again heated to 120 °C for 15 minutes to allow for the removal of the SEM protecting group. 12.5 ml of 25 mM ammonium formate buffer (from vial 4) was then added to the reaction mixture with stirring. The reactor needle was lowered to the bottom of the reactor, and the contents were pushed over the first HLB cartridge to trap the crude product. The crude product was then eluted into tube 2 (containing ammonium formate buffer, 3.5 ml, 25 mM) with acetonitrile (1 ml, from vial 6). The contents of tube 2 were then loaded onto the HPLC injection loop (5 ml) and injected onto the HPLC column for purification using eluent A. The product radio peak appeared at 10-12 minutes and was cut into the large dilution reservoir. The contents of the dilution were then stirred and passed over the second HLB cartridge into the waste. The product, which was trapped on the HLB cartridge, was washed with water (4 ml, from vial 14), after which it was eluted with ethanol (0.5 ml) into the product collection vial. The product was finally reconstituted with PBS (4.5 ml). The final tracer solution was pushed out into a sterile product delivery vial to afford a solution of [¹⁸F] olaparib (5.4 ± 1.6 %AY; 9.3 ± 3.3 %RCY; synthesis time 90 min) in PBS with 10% ethanol.

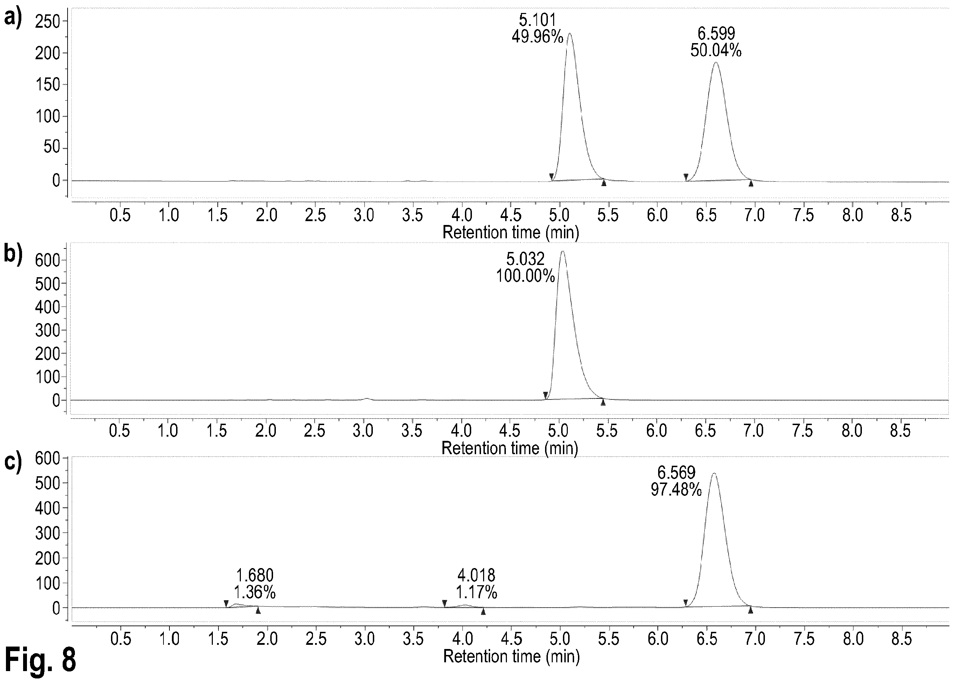

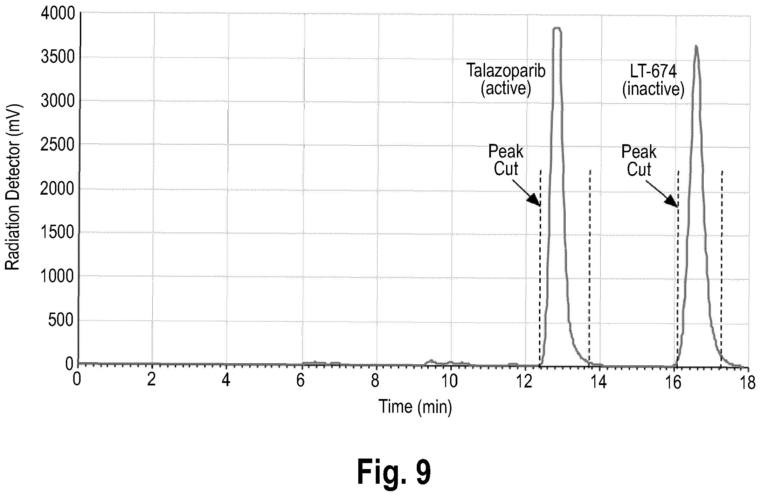

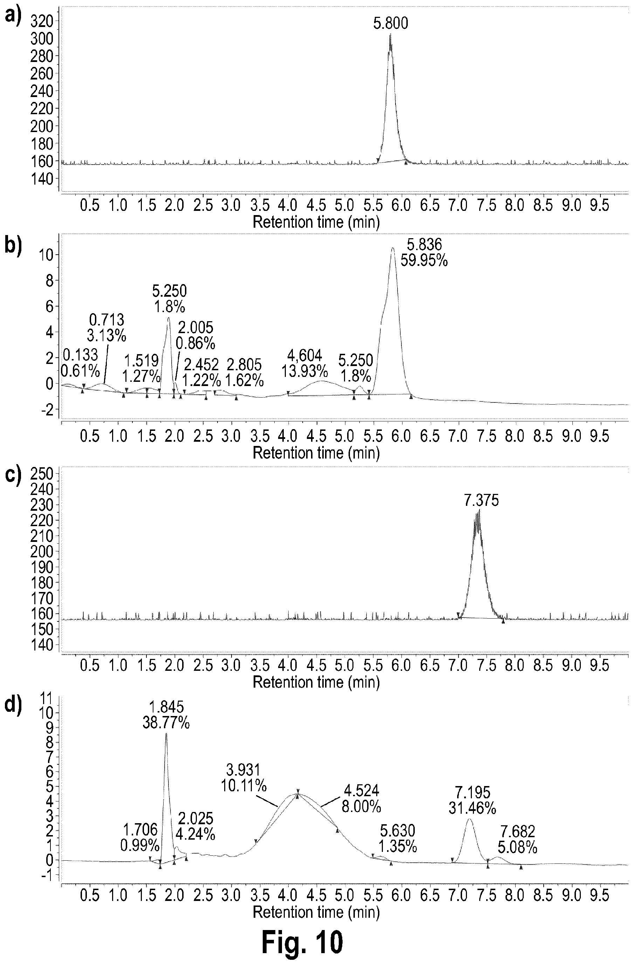

Figs. 8 to 10 show RadioHPLC of the different compounds.

### In vitro Binding studies

The *in vitro* Binding studies involved the use of cell cultures: Human breast carcinoma cells (HCC1937, ACC513) were purchased from the German Collection of Microorganisms and Cell Cultures (DSMZ GmbH, Braunschweig, Germany) and cultured in Roswell Park Memorial Institute (RPMI) 1640 medium supplemented with 16 % fetal calf serum (FCS), 100 U/ml penicillin and 100 µg/ml streptomycin at 37°C under humid 5 % CO2 atmosphere. Absence of mycoplasma infection was confirmed by PCR analysis in monthly intervals.

### In vitro radiotracer uptake

HCC1937 cells (0.2 x 106) were incubated in 96-well filter plates (MADVN6550, Merck Millipore, Darmstadt, Germany) with 60 µl of a 0.4 MBq/ml radiotracer solution containing either 2.5 µl DMSO as vehicle, 2.5 µl 10 mM olaparib or 2.5 µl talazoparib to a final concentration of 25 µM for blocking. After 30 min of incubation at 37°C, the cells were washed by vacuum filtration of medium through the plate (2x100 µl followed by 2x200 µl), the filters were transferred into tubes using a commercial punch kit (MAMP09608, Merck) and measured in a gamma counter (Wizard 2, Perkin-Elmer, Waltham, MA, USA). Experiments were performed in triplicates and the uptake was quantified as percent of added activity.

### Competition assays

HCC1937 cells (0.2 x 106) were incubated in 96-well filter plates (MADVN6550, Merck Millipore, Darmstadt, Germany) with 40 µl of a 0.4 MBq/ml radiotracer and 20 µl of a 1:2 serial dilution of either talazoparib or olaparib starting with a final concentration of 1µM. After 30 min of incubation at 37°C, the cells were washed by vacuum filtration of medium through the plate (2x100 µl followed by 2x200 µl), the filters were transferred into tubes using a commercial punch kit (MAMP09608, Merck) and measured in a gamma counter (Wizard 2, Perkin-Elmer, Waltham, MA, USA). Experiments were performed in triplicates and the uptake was quantified as percent of added activity.

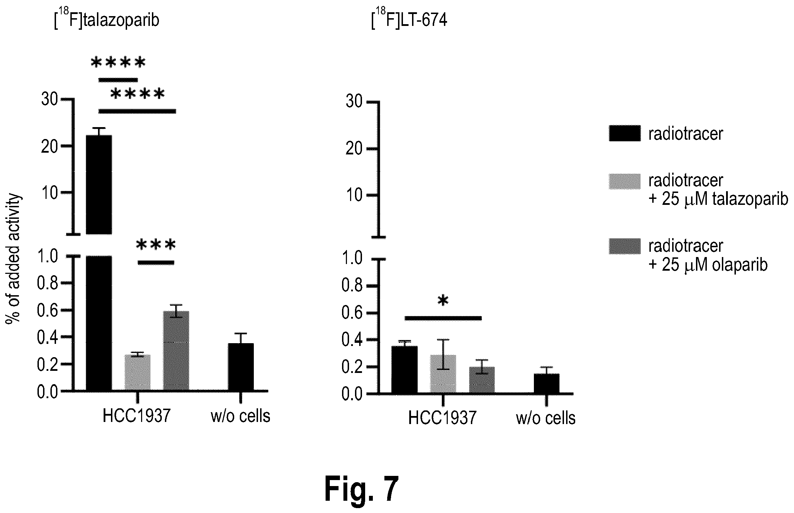

The results of the *in vitro* radiotracer uptake are shown in Fig. 7. Is clearly derivable that [¹⁸F] uptake is in high amounts, which also demonstrates the potential *in vivo* use of the present compounds of formula 10a and 10b.

### Summary

The present inventors have shown the successful synthesis of [¹⁸F] talazoparib and of its derivatives. In addition, the general feasibility of [¹⁸F] talazoparib and [¹⁸F] talazoparib derivatives for imaging, particularly PET imaging, has been shown. The disclosed successful first synthesis of [¹⁸F] talazoparib is not only of great interest for nuclear medicine, but also proves the crucial impact of copper-mediated radiofluorination for the development of novel radiotracers.

## Claims

1. A compound of the formula 10a and/or 10b 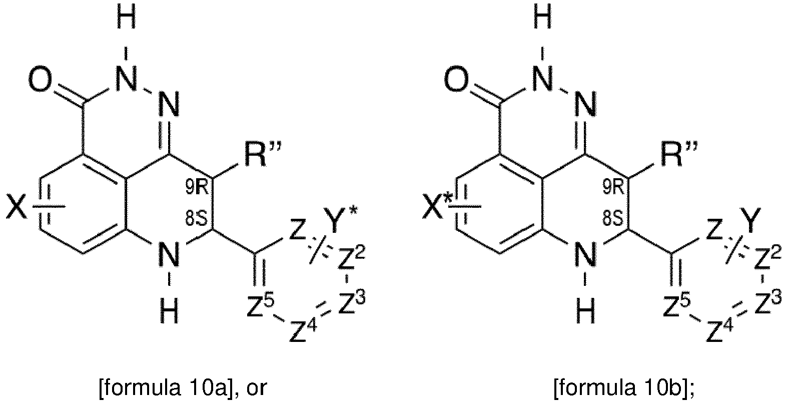 or
a precursor of the formula 5c 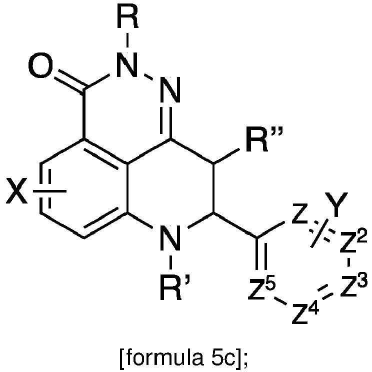 wherein
R and R' are amine protecting groups;
R" is a heteroaromatic or an aromatic residue of a molecular weight of 120 g/mol or less;
X and Y are independently selected from the group consisting of H, one or more leaving groups for [¹⁸F] fluorination, and a saturated or unsaturated alkyl residue of a molecular weight of 80 g/mol or less, provided that at least one leaving group for [¹⁸F] fluorination is present;
X* and Y* are independently selected from the group consisting of H, [¹⁸F], one or more leaving groups for [¹⁸F] fluorination and a saturated or unsaturated alkyl residue of a molecular weight of 80 g/mol or less, provided that at least one [¹⁸F] is present;
z and Z²⁻are independently selected from the group consisting of C and N; an enantiomeric ratio (er) at a position C8 and/or a position C9 of the precursor is ≥ 1.

2. A method for producing a compound of the formula 10a and/or 10b 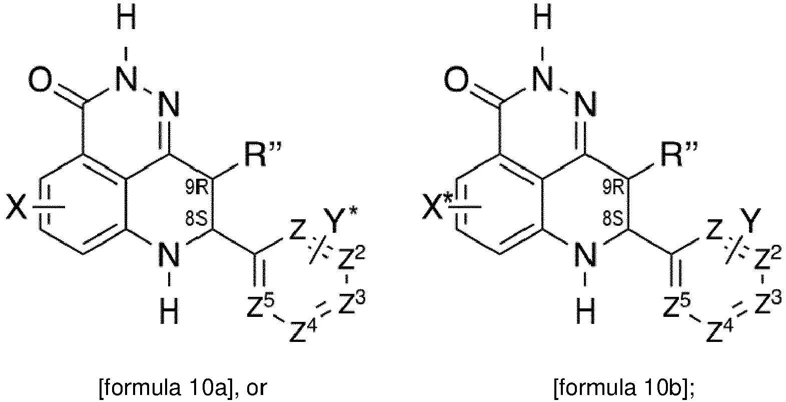 comprising the steps of:
(A) providing a precursor of the formula 5c 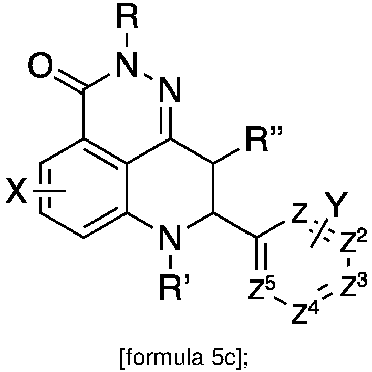
(B) reacting the precursor of the formula 5c with an [¹⁸F] radiofluorination agent to obtain a [¹⁸F] fluorinated compound of the formula 8a and/or 8b, 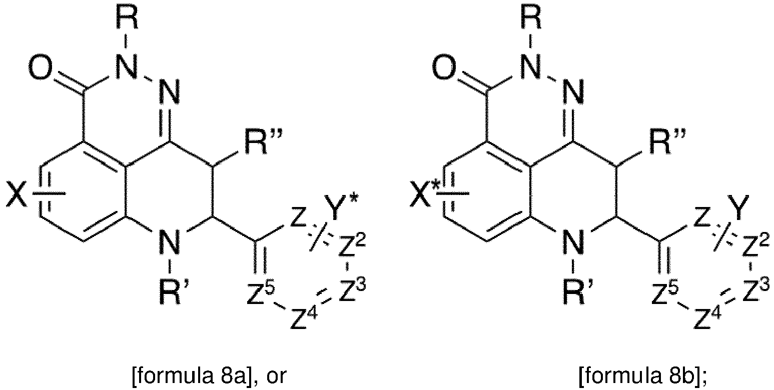
(C) de-protecting the [¹⁸F] fluorinated compound of the formula 8a and/or 8b to obtain a compound of the formula 9a and/or 9b 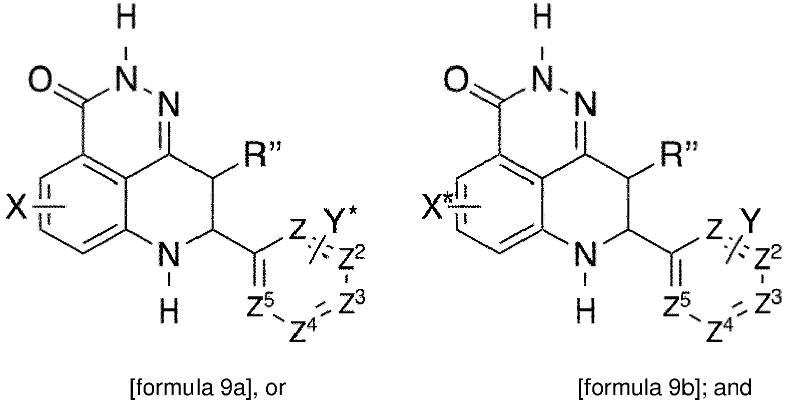
(D) subjecting the compound of the formula 9a and/or 9b to purification and optionally enantiomeric resolution to obtain the compound of the formula 10a and/or 10b,
wherein
R and R' are amine protecting groups;
R" is a heteroaromatic or an aromatic residue of a molecular weight of 120 g/mol or less;
X and Y are independently selected from the group consisting of H, one or more leaving groups for [¹⁸F] fluorination, or a saturated or unsaturated alkyl residue of a molecular weight of 80 g/mol or less, provided that at least one leaving group for [¹⁸F] fluorination is present;
X* and Y* are independently selected from the group consisting of H, [¹⁸F], one or more leaving groups for [¹⁸F] fluorination and a saturated or unsaturated alkyl residue of a molecular weight of 80 g/mol or less, provided that at least one [¹⁸F] is present;
z and z²⁻⁵ are independently selected from the group consisting of C and N;
an enantiomeric ratio (er) at a position C8 and/or a position C9 of the precursor is ≥ 1.

3. The compound of claim 1 or the method of claim 2, wherein the amine protecting group at R and R' is independently selected from the group consisting of Trimethylsilylethoxymethyl, *t*-Butyl carbamate, Benzyloxy carbamate, and Methoxymethyl acetal.

4. The compound of claim 1 or 3 or the method of any of claims 2-3, wherein the heteroaromatic residue is selected from the group consisting of Me-1,2,4-triazol-5-yl, 4-Me-1,2,4-triazol-3-yl, 4-pyridyl, 3-pyridyl, 2-pyridyl, 1-Me-imidazol-2-yl, 1-Me-pyrazol-5-yl, wherein the aromatic residue is phenyl.

5. The compound of any of claims 1 or 3-4 or the method of any of claims 2-4, wherein z is N and z²⁻⁵ are C; z² is N and z, z³⁻⁵ are C; or z³ is N and z, z², z⁴⁻⁵ are C.

6. The compound of any of claims 1 or 3-5 or the method of any of claims 2-5, wherein the leaving group for [¹⁸F] fluorination is selected from the group consisting of Boronic acid, Boronic acid ester, lodonium salt, lodonium ylid, Trialkylstannane, Nitro, Trialkyl ammonium salts, and Sulfonate esters, such as OTs, OMs, ONs, OBs, or OTf.

7. The compound of any of claims 1 or 3-6 or the method of any of claims 2-6, wherein the radiofluorination agent is a nucleophilic [¹⁸F] radiofluorination agent, preferably wherein the nucleophilic [¹⁸F] radiofluorination agent is [¹⁸F] TBAF.

8. The compound of any of claims 1 or 3-7 or the method of any of claims 2-7, wherein the compound of the formula 10a is [18F] talazoparib, and/or
the compound of formula 10b is (8S,9R)-5-[¹⁸F]-8-(4-fluorophenyl)-2,7,8,9-tetrahydro-9-(1-methyl-1H-1,2,4-triazol-5-yl)- 3H-pyrido[4,3,2-de]phthalazin-3-one.

9. The method of claim 2, including the step of preparing the precursor of the formula 5c by (i) reacting a compound of the formula 5d and/or 5e 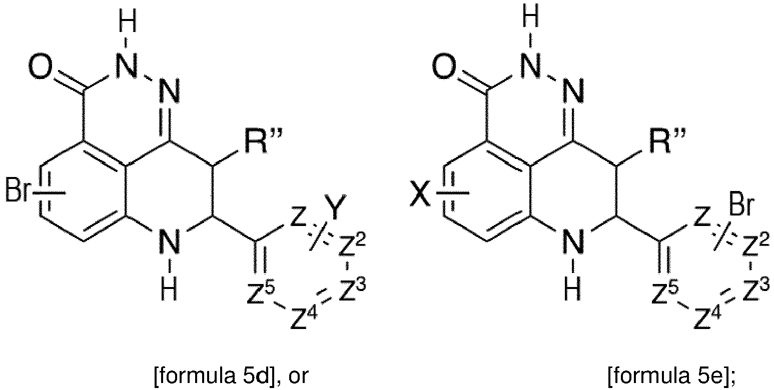 with a trimethylsilylethoxymethyl salt, preferably trimethylsilylethoxymethyl chloride; and
(ii) derivatizing to the corresponding boronic acid pinacol esters of the formula 7d and/or 7e 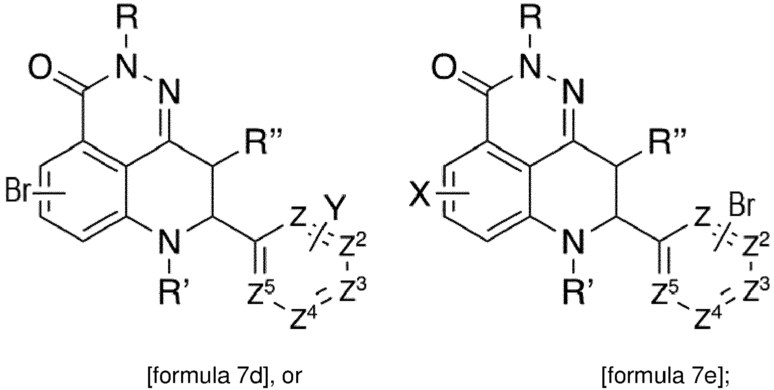 wherein R' is trimethylsilylethoxymethyl, R" is trimethylsilylethoxymethyl or H, and the leaving group for [¹⁸F] fluorination is boronic acid pinacol ester or tributylstannane.

10. The method of claim 9, wherein the compound of the formula 5d is (±)-5-Fluoro-8-(4-bromophenyl)-2,7,8,9-tetrahydro-9-(1-methyl-1*H*-1 ,2,4-triazol-5-yl)-3H-pyrido[4,3,2-de]phthalazin-3-one, and/or the compound of the formula 5e is (±)-5-bromo-8-(4-fluorophenyl)-2,7,8,9-tetrahydro-9-(1-methyl-1H-1,2,4-triazol-5-yl)-3H-pyrido[4,3,2-de]phthalazin-3-one.

11. The method of any of claims 2-10, wherein purification of the compound of the formula 9a and/or 9b of step (D) includes subjecting the compound of the formula 9a and/or 9b to solid phase extraction with a sorbent for polar analytes to obtain a purified compound of the formula 9a and/or 9b.

12. The method of claim 11, wherein enantiomeric resolution of the compound of the formula 9a and/or 9b of step (D) includes subjecting the purified compound of the formula 9a and/or 9b to one or more of chiral NPLC, chiral SFC, and chiral HPLC to obtain the compound of the formula 10a and/or 10b.

13. The method of any of claims 2-11, wherein the step (B) of reacting the precursor of the formula 5c with an [¹⁸F] radiofluorination agent includes copper mediated radiofluorination chemistry that is optimized with respect to the radiochemical yield of the (±)¹⁸F fluorinated compound of the formula 8a and/or 8b.

14. Method for preparing a diagnostic agent comprising the steps of:
providing the compound of formula 10a and/or 10b according to any of claims 1 or 3-8; and
formulating the compound with a pharmaceutically acceptable carrier and/or a solvent and, if applicable, further pharmaceutical excipients,
preferably wherein the diagnostic agent is designated for a use within the positron emission tomography.

15. The compound of the formula 10a and/or 10b according to any of claims 1 or 3-8 for use in surgery.
